# EUROPEAN PATENT SPECIFICATION

(11) **EP 4 499 627 B1**
(45) Date of publication and mention of the grant of the patent: **02.12.2026**
(21) Application number: 22719858.7
(22) Date of filing: 30.03.2022
(51) Int. Cl.: C07D 401/14, A61P 35/00, A61K 31/4709

(54) **NEW FORMS OF N-(5-((6,7-DIMETHOXYQUINOLIN-4-YL)OXY)PYRIDIN-2-YL)-1-PROPYL-4-(2,2,2-TRIFLUOROETHOXY)-1H-PYRAZOLE-3-CARBOXAMIDE HYDROCHLORIDE**
NEUE FORMEN VON N-(5-(6,7-DIMETHOXYCHINOLIN-4-YL)OXY)PYRIDIN-2-YL)-1-PROPYL-4-(2,2,2-TRIFLUORETHOXY)-1H-PYRAZOL-3-CARBOXAMIDHYDROCHLORID
NOUVELLES FORMES DE CHLORHYDRATE DE N-(5-((6,7-DIMÉTHOXYQUINOLIN-4-YL)OXY)PYRIDIN-2-YL)-1-PROPYL-4-(2,2,2-TRIFLUOROÉTHOXY)-1H-PYRAZOLE-3-CARBOXAMIDE

(43) Date of publication of application: 05.02.2025
(73) Proprietor: Qurient Co., Ltd., Seongnam-si, Gyeonggi-do 13487 (KR)
(72) Inventor: NAM, Kiyean, Seongnam-si, Gyeonggi-do 13554 (KR); KIM, Jaeseung, Seoul 06095 (KR); JEON, Borami, Suwon-si, Gyeonggi-do 16539 (KR)
(74) Representative: Engelhard, Markus
(86) International application number: PCT/EP2022/058450
(87) International publication number: WO 2023/186294

(56) References cited:
- WO-A1-2019/229251

## Description

The present invention relates to new forms of inhibitors of Axl/Mer receptor tyrosine kinase and CSF1R (colony stimulating factor 1 receptor), and in particular to new forms of N-(5-((6,7-dimethoxyquinolin-4-yl)oxy)pyridin-2-yl)-1-propyl-4-(2,2,2-trifluoro-ethoxy)-1H-pyrazole-3-carboxamide hydrochloride.

Axl/Mer receptor tyrosine kinase (Axl/Mer RTK) is a member of the TAM (tyrosine, Axl, Mer) receptor tyrosine kinases. Such TAM receptor tyrosine kinases are characterized by an extra cellular domain consisting of two immunoglobulin-like domains followed by two fibronectin type-3-like domains. Activation of the Axl/Mer-pathway occurs by the cognate protein ligand, i. e. growth arrest specific 6 (Gas6) and protein S (Pros1), respectively.

A number of inhibitors of such TAM receptor tyrosine kinases have been described, for example in Myers et al., 2019, Mol. Cancer, 18, 94; https: //doi.org/10.1186/s12943-019-1022-2.

CSF1R is known to regulate the differentiation of myeloid progenitors into heterogeneous populations of monocytes, macrophages, dendritic cells (DC) and bone-resorbing osteoclasts. In addition, activated CSF1R promotes the survival, proliferation, differentiation and chemotaxis of differentiated macrophages (Geissmann F et al., Science. 2010 Feb 5; 327(5966):656-61).

Based on role of CSF1R in immune cells, various approaches targeting either the CSF1R or its ligands is developing against immunotherapy and cancers and currently it in clinical stage.

A number of such inhibitors, including the compound N-(5-((6,7-dimethoxyquinolin-4-yl)oxy)pyridin-2-yl)-1-propyl-4-(2,2,2-trifluoroethoxy)-1H-pyrazole-3-carboxamide, also referred to as "Q702", have been described in WO 2019/229251.

There is a continuing need in the art to find an improved suitable inhibitor of Axl/Mer receptor tyrosine kinase and CSF1R that affords a high bioavailability, a good solubility and a good stability.

These objects are solved by Compound N-(5-((6,7-dimethoxyquinolin-4-yl)oxy)pyridin-2-yl)-1- propyl-4-(2,2,2-trifluoroethoxy)-1H-pyrazole-3-carboxamide hydrochloride having the formula 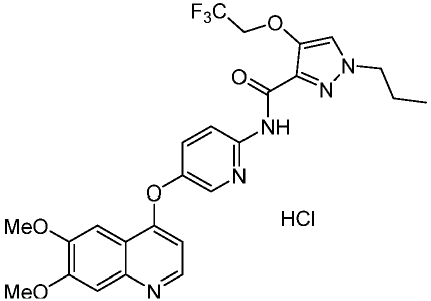 and having at least six peaks from a first set of peaks in an X-ray Powder Diffraction (XRPD) spectrum obtained by irradiation with Cu-Kα - radiation (Cu-Kα), said first set of peaks being:
8.2 ° 2θ, 9.9° 2θ, 11.4 ° 2θ, 15.2° 2θ, 15.4 ° 2θ, 19.9 ° 2θ, 21.4 ° 2θ, 21.7 ° 2θ, 22.9 ° 2θ, 23.3 ° 2θ, 25.5 ° 2θ, 25.7 ° 2θ, 25.9 ° 2θ, 28.2 ° 2θ, and 28.7 ° 2θ, ± 0.2 ° 2θ;
such compound herein also being designated as ("form A" of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride).

In one embodiment, the compound has at least two peaks from a second set of peaks in an X-ray Powder Diffraction (XRPD) spectrum obtained by irradiation with Cu-Kα - radiation (Cu-Kα), said second set of peaks being:
3.8 ° 2θ, 7.6 ° 2θ, 10.7 ° 2θ, 12.9 ° 2θ, 16.5 ° 2θ, 17.2 ° 2θ, 24.6 ° 2θ, 24.8 ° 2θ, 29.5 ° 2θ, 29.9 ° 2θ, and 31.1 ° 2θ, ± 0.2 ° 2θ;
wherein, preferably, said compound has at least three peaks or at least four peaks from said second set of peaks, such compound herein also being designated as ("form A" of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride).

In one embodiment, said compound has all of the peaks from said first set of peaks and/or from said second set of peaks, such compound herein also being designated as ("form A" of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride).

In one embodiment, the compound has an XRPD spectrum obtained by irradiation with Cu-Kα - radiation (Cu-Kα) and as shown hereafter: 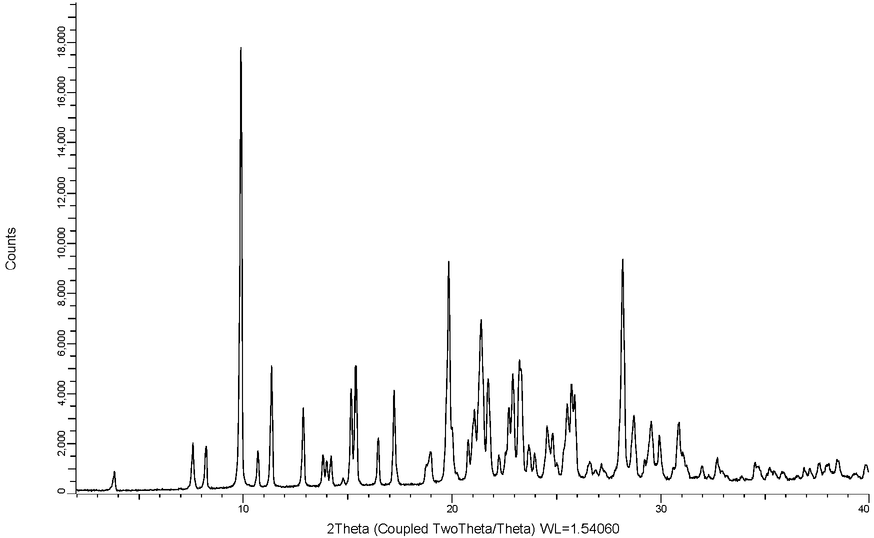 , such compound herein also being designated as ("form A" of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride)

In one embodiment, the compound has a differential scanning calorimetry (DSC) thermogram showing an in the range of from 115 °C to 150 °C, preferably in the range of from 135 °C to 148 °C, more preferably in the range of from 135 °C to 146 °C, even more preferably in the range of from 140 °C to 146 °C, even more preferably in the range of from 144 °C to 146 °C, even more preferably at approximately 145 °C, most preferably at approximately 145.8 °C.

In one embodiment, the compound has a differential scanning calorimetry (DSC) thermogram as shown hereafter:

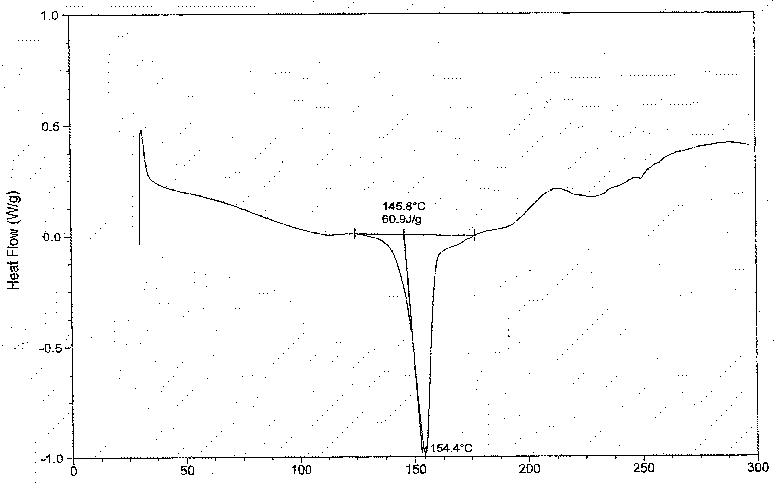

In one embodiment, the compound is produced by a method comprising the steps:
- Providing, in any order, a defined amount of N-(5-((6,7-dimethoxyquinolin-4-yl)oxy)pyridin-2-yl)-1- propyl-4-(2,2,2-trifluoroethoxy)-1H-pyrazole-3-carboxamide free base and a defined amount of hydrochloric acid, such that said free base and said hydrochloric acid are provided in a stoichiometric ratio of 1:1;
- dissolving the freebase in a suitable solvent or solvent mixture, selected from methanol, ethanol, tetrahydrofuran (THF), acetone, water and a mixture of water with any of methanol, ethanol, tetrahydrofuran (THF) and acetone; and, optionally, adding 1 - 5 reaction volumes of water;
- adding approximately half of the defined amount of said hydrochloric acid;
- Adding a seeding amount of form A of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride, such form A being as defined herein, e.g. in any of claims 1 - 4; thereby promoting formation and precipitation of form A;
- Adding the other half of the defined amount of said hydrochloric acid over a period of from 30 min to 5 h;
- Stirring for a defined period of from 30 min to 2 h, preferably from 30 min to 1 h;
- Adding water in an amount of 5 to 15 reaction volumes, preferably approximately 10 reaction volumes, over a period of 1 - 20 h, preferably over a period of 2 - 10 h;.
optionally isolating precipitated form A, preferably by filtration, sieving or solvent evaporation.

In a further aspect, the present invention also relates to a method for making the compound as defined herein, said method comprising the steps:
- Providing, in any order, a defined amount of N-(5-((6,7-dimethoxyquinolin-4-yl)oxy)pyridin-2-yl)-1- propyl-4-(2,2,2-trifluoroethoxy)-1H-pyrazole-3-carboxamide free base and a defined amount of hydrochloric acid, such that said free base and said hydrochloric acid are provided in a stoichiometric ratio of 1:1;
- dissolving the freebase in a suitable solvent or solvent mixture, selected from methanol, ethanol, tetrahydrofuran (THF), acetone, water and a mixture of water with any of methanol, ethanol, tetrahydrofuran (THF) and acetone; and, optionally, adding 1 - 5 reaction volumes of water;
- adding approximately half of the defined amount of said hydrochloric acid;
- Adding a seeding amount of form A of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride, such form A being as defined herein, e.g. in any of claims 1 - 4; thereby promoting formation and precipitation of form A;
- Adding the other half of the defined amount of said hydrochloric acid over a period of from 30 min to 5 h;
- Stirring for a defined period of from 30 min to 2 h, preferably from 30 min to 1 h;
- Adding water in an amount of 5 to 15 reaction volumes, preferably approximately 10 reaction volumes, over a period of 1 - 20 h, preferably over a period of 2 - 10 h;.
optionally isolating precipitated form A, preferably by filtration, sieving or solvent evaporation.

In a further aspect, the present invention also relates to a pharmaceutical composition comprising at least one compound as defined herein, together with at least one pharmaceutically acceptable carrier, excipient and/or diluent.

In one embodiment of the pharmaceutical composition, it further comprises at least one other pharmaceutically active agent.

In a further aspect, the present invention also relates to a compound or pharmaceutical composition as defined herein, for use in the treatment of a disorder selected from hyperproliferative disorders, inflammatory disorders and neurodegenerative disorders.

In one embodiment of such compound or pharmaceutical composition for use, said hyperproliferative disorder is a cancer, preferably a cancer selected from adenocarcinoma, acoustic neuroma, acute lymphoblastic leukemia, acute myeloid leukemia, adrenocortical carcinoma, aids-related cancers, aids-related lymphoma, anal cancer, appendix cancer, astrocytomas, atypical teratoid/rhabdoid tumor, ampullary carcinoma, basal cell carcinoma, bile duct cancer, bladder cancer, bone cancer, osteosarcoma and malignant fibrous histiocytoma, brain stem glioma, brain tumor, central nervous system atypical teratoid/rhabdoid tumor, craniopharyngioma, ependymoblastoma, ependymoma, medulloblastoma, medulloepithelioma, pineal parenchymal tumors of intermediate differentiation, supratentorial primitive neuroectodermal tumors and pineoblastoma, brain and spinal cord tumors, breast cancer, urachal tumors, burkitt lymphoma, carcinoid tumor, choroidal melanoma, gastrointestinal cancer, central nervous system lymphoma, cervical cancer, corpus cancer, chordoma, chronic lymphocytic leukemia, chronic myelogenous leukemia, chronic myeloproliferative disorders, colon cancer, colorectal cancer, cutaneous t-cell lymphoma, desmoid tumor, mycosis fungoides, endometrial cancer, esophageal cancer, esthesioneuroblastoma, Ewing sarcoma family of tumors, extracranial germ cell tumor, extragonadal germ cell tumor, extrahepatic bile duct cancer, ear tumors, intraocular melanoma, retinoblastoma, gallbladder cancer, gastric cancer, gastrointestinal carcinoid tumor, gastrointestinal stromal tumor, gastrointestinal stromal cell tumor, gynecologic tumors, ovarian germ cell tumor, gestational trophoblastic tumor, glioma, gallbladder carcinomas, hairy cell leukemia, head and neck cancer, heart cancer, hepatocellular cancer, histiocytosis, hypopharyngeal cancer, hematologic neoplasias, islet cell tumors (endocrine pancreas), renal cell cancer, kidney cancer, langerhans cell histiocytosis, laryngeal cancer, leukemia, lip and oral cavity cancer, liver cancer, lung cancer, non-small cell lung cancer, small intestinal tumors, small cell lung cancer, hodgkin lymphoma, non-hodgkin lymphoma, primary central nervous system lymphoma, macroglobulinemia, malignant fibrous histiocytoma of bone and osteosarcoma, melanoma, merkel cell carcinoma, mesothelioma, metastatic squamous neck cancer with occult primary, spinalioms, multiple endocrine neoplasia syndromes, myelodysplastic syndromes, myelodysplastic/myeloproliferative neoplasms, myeloid leukemia, multiple myeloma, myeloproliferative disorders, nasal cavity and paranasal sinus cancer, nasopharyngeal cancer, neuroblastoma, oral cavity cancer, oropharyngeal cancer, osteosarcoma and malignant fibrous histiocytoma of bone, ovarian cancer, ovarian epithelial cancer, ovarian low malignant potential tumor, oligodendroglioma, plasmacytomas, pancreatic cancer, papillomatosis, parathyroid cancer, penile cancer, pharyngeal cancer, pituitary tumor, plasma cell neoplasm/multiple myeloma, pleuropulmonary blastoma, prostate cancer, rectal cancer, renal cell cancer, transitional cell cancer, respiratory tract cancer, rhabdomyosarcoma, salivary gland cancer, sarcoma, skin testis cancer, ewing sarcoma, kaposi sarcoma, uterine sarcoma, non-melanoma skin cancer, melanoma skin cancer, skin carcinoma, small intestine cancer, soft tissue sarcoma, squamous cell carcinoma, squamous neck cancer, stomach cancer, soft tissue tumors, testicular cancer, throat cancer, thymoma and thymic carcinoma, thyroid cancer, transitional cell cancer of the renal pelvis and ureter, trophoblastic tumor, testicle cancer, gestational cancer, urologic tumors, ureter and renal pelvis cancer, urethral cancer, urothelial carcinoma, uterine cancer, vaginal cancer, vulvar cancer, waldenström macroglobulinemia and wilms tumor, tumors that cause effusions in potential spaces of the body, pleural effusions, pericardial effusions, peritoneal effusion aka ascites, giant cell tumor (GCT), GCT of bone, pigmented villonodular synovitis (PVNS), tenosynovial giant cell tunor (TGCT), TGCT of tendon sheath (TGCT-TS).

In one embodiment of such compound or pharmaceutical composition for use, said inflammatory disorder is selected from osteoarthritis, inflammatory bowel syndrome, tramsplant rejection, systemic lupus erythematosis, ulcerative colitis, crohn's disease, chronic obstructive pulmonary disease, emphysema, Kawasaki's Disease, hemophagocytic syndrome (macrophage activation syndrome), multicentric reticulohistiocytosis, atherosclerosis, primary progressive multiple sclerosis, tenpsy Type I diabetes, Type Il diabetes, insulin resistance, hyperglycemia, obesity, lipolysis, hypcreosinophilia, osteoporosis, increased risk of fracture, Paget's disease, hypercalcemia, infectionmediated osteolysis (e.g. osteomyelitis), peri-prosthetic or wear-debris-mediated osteolysis, endometriosis, inflammatory pain, chronic pain, and bone pain.

In one embodiment of such compound or pharmaceutical composition for use, said neurodegenerative disorder is selected from Binswanger type dementia, prosencephaly, microcephaly, cerebral palsy, congenital hydrocephalus, abdominal dropsy, progress supranuclear palsy, glaucoma, Wilson disease, Alzheimer's disease and other dementias, Parkinson's disease (PD) and PD-related disorders, multi infarct dementia, Frontotemporal dementia, pseudo-dementia, Prion disease, Motor neuron diseases, Huntington's disease, spinocerebellar ataxia, and spinal muscular atrophy.

In one embodiment of such compound or pharmaceutical composition for use, wherein said use is in combination with another pharmaceutically active drug or therapy, in particular radiation therapy, chemotherapy agents, targeted drugs and immune check point inhibitor drugs.

Also disclosed, but not forming part of the invention is a method of treatment of a disease selected from hyperproliferative disorders, inflammatory disorders and/or neurodegenerative disorders, said method comprising the administration of a compound as defined herein, or the pharmaceutical composition as defined herein, to a patient in need thereof.

In yet a further aspect, the present invention relates to a use of a compound as defined herein, for the manufacture of a medicament for the treatment of a disease selected from hyperproliferative disorders, inflammatory disorders and/or neurodegenerative disorders.

In both the aforementioned aspects, the disease selected from hyperproliferative disorders, inflammatory disorders and/or neurodegenerative disorders, the compound and the pharmaceutical composition are as defined herein.

As used herein, the term "Q702" refers to compound N-(5-((6,7-dimethoxyquinolin-4-yl)oxy)pyridin-2-yl)-1-propyl-4-(2,2,2-trifluoroethoxy)-1H-pyrazole-3-carboxamide. Likewise, the term "Q702 salt" or "Q702 HCl" or "Q702 HCl salt", as used herein, refers to a salt in general or an HCl form or an HCl salt, respectively, of such compound.

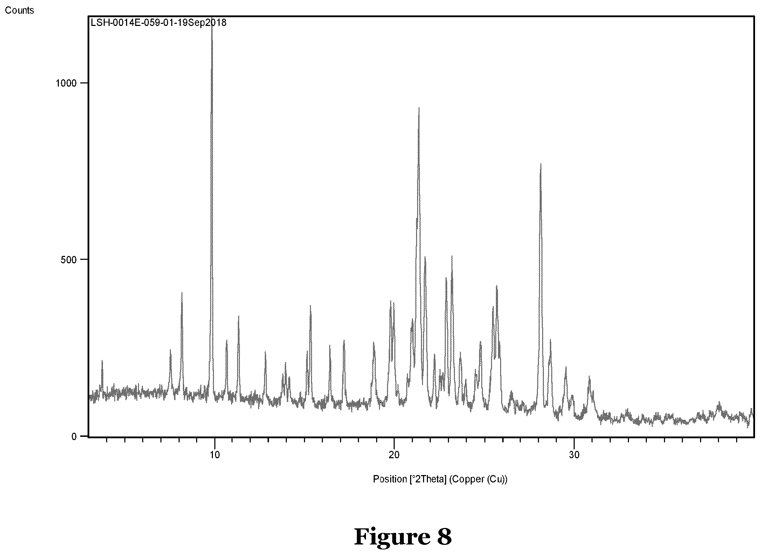

The term "form A of Q702 HCl", as used herein, refers to a specific crystal form of Q702 HCl, as defined by various peaks in an XRPD spectrum, or the entire XRPD spectrum thereof. Such peaks and spectra of such form A are outlined and described herein, for example in any of claims 1 - 4, or in figure 8.

The term "seeding amount", or "seeding amount of form A", as used herein, is meant to refer to any amount of form A of Q702 HCl that is suitable and sufficient to act as a nucleation site or point for crystallization of form A of Q702 HCl, or to promote the growth of more crystals of form A of Q702 HCl.

A person skilled in the art will be able to determine and identify such amount, in view of the reaction conditions and the reaction scale chosen in each case. Seeding crystallisation is for example reviewed in Parambil, 2017, "Engineering Crystallography: From Molecule to Crystal to Functional Form (pp.235-245)" (DOI:10.1007/978-94-024-1117-1 13)

Moreover, reference is made to the enclosed figures, wherein
Figure 1 shows XRPD spectra of the different Q702 HCl salt forms identified in the course of the present invention, namely forms A - F (from top to bottom) in Figure 1a, forms G - L (from top to bottom) in figure 1b, and forms M - R (from top to bottom) in figure 1c.
Figure 2 shows the XRPD spectrum of amorphous Q702 HCl prepared by rotary evaporation and lyophilisation.
Figure 3 shows an XRPD spectrum of Q702 HCl salt form C.
Figure 4 shows TGA analysis data for Q702 HCl salt form C (open pan).
Figure 5 shows TGA analysis data for Q702 HCl salt form C (closed pan).

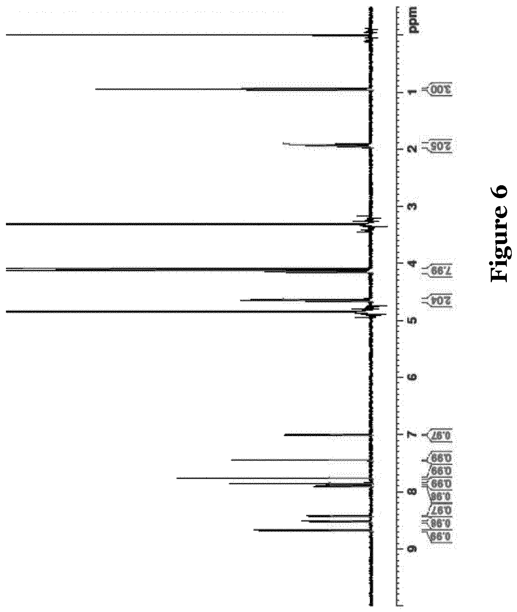

Figure 6 shows an ¹H NMR spectrum of Q702 HCl salt form C.
Figure 7 shows DVS data for Q702 HCl salt form C.
Figure 8 shows an XRPD spectrum of Q702 HCl salt form A.
Figure 9 shows TGA analysis data for Q702 HCl salt form A (closed pan).
Figure 10 shows TGA analysis data for Q702 HCl salt form A (open pan).
Figure 11 shows an ¹H NMR spectrum of Q702 HCl salt form A.
Figure 12 shows DVS data for Q702 HCl salt form A.
Figure 13 shows a DVS kinetics plot for Q702 HCl salt form A.
Figure 14 shows XRPD spectra for Q702 HCl salt form A prior to (top) and after DVS analysis (bottom).
Figure 15 shows XRPD spectra for Q702 HCl salt form A prior to (top) and after (bottom) exposure to 40 °C and 75%RH.
Figure 16 shows an ¹H NMR spectrum of Q702 HCl salt form A after 15 days exposure to 40 °C and 75%RH.
Figure 17 shows the results of the crystallization experiment 1 of example 3, with the top four traces showing the XRPD-spectra of the different stages of the reaction and the lower trace (labelled as "PS02379-129-N-P.brmi") being a reference trace for form A of Q702.
Figure 18 shows the results of the crystallization experiment 2 of example 3, with the top four traces showing the XRPD-spectra of the different stages of the reaction and the lower trace (labelled as "PS02379-129-N-P.brmi") being a reference trace for form A of Q702.
Figure 19 shows the results of the crystallization experiment 3 of example 3, with the top four traces showing the XRPD-spectra of the different stages of the reaction and the lower trace (labelled as "PS02379-129-N-P.brmi") being a reference trace for form A of Q702.
Figure 20 shows the results of the crystallization experiment 4 of example 3, with the top four traces showing the XRPD-spectra of the different stages of the reaction and the lower trace (labelled as "PS02379-129-N-P.brmi") being a reference trace for form A of Q702.
Figure 21 shows the results of the crystallization experiment 5 of example 3, with the top four traces showing the XRPD-spectra of the different stages of the reaction and the lower trace (labelled as "HCl Pat C.txt") being a reference trace for form A of Q702.
Figure 22 shows the results of the crystallization experiment 6 to 9 of example 3, with the top four traces showing the XRPD-spectra at different stages of the experiment as described, and the lower trace showing a reference trace for form A, labelled as "PS02379-129-N-P.brmi".

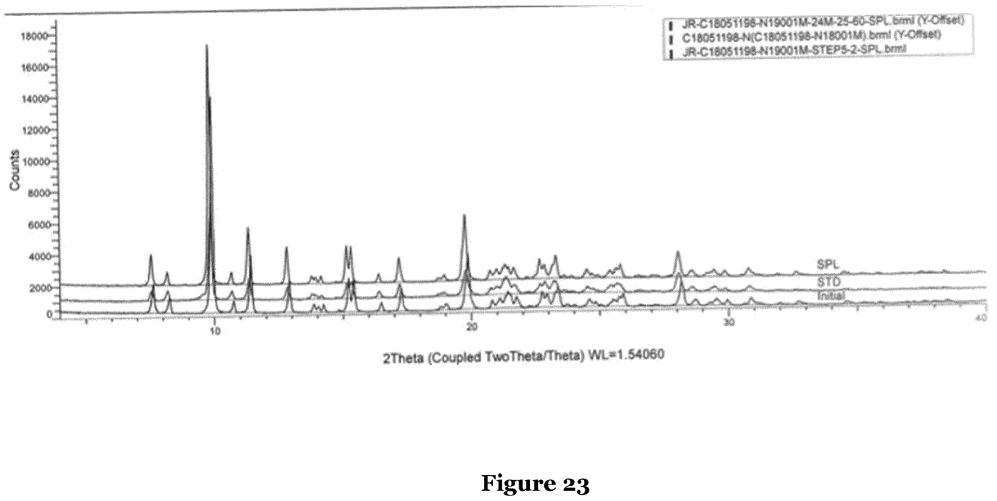

Figure 23 shows on overlay of XRPD spectrum of Q702 HCl salt form A after 24 months exposure to 25 °C and 60%RH, with the top traces showing the XRPD-spectra at 24 months exposure to 25 °C and 60%RH, and the middle trace (labelled as "C18051198-N(C18051198-N18001M)") being a reference trace for form A of Q702, and the lower trace showing form A of Q702 as initially prepared, i. e. without 24-months' exposure.

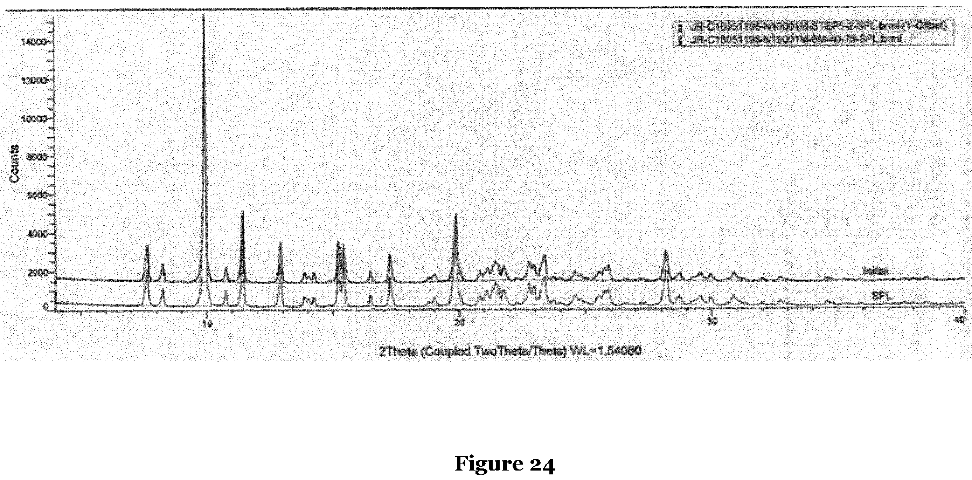

Figure 24 shows on overlay of XRPD spectrum of Q702 HCl salt form A after 6 months exposure to 40 °C and 75%RH, with the top traces showing the XTPD-spectra at 24 months exposure to 25 °C and 60%RH, and the lower trace showing form A of Q702 as initially prepared, i. e. without 6-months' exposure.
Figure 25 shows an overlay of mean plasma concentrations of Q702 in mice after oral dosing of Q702 HCl Form A and Q702 free base, both at 30.0 mg/kg.

Furthermore, reference is made to the following examples, which are given to illustrate, not to limit the present invention.

### Examples

### Example 1

### Screening for the best salt

The compound N-(5-((6,7-dimethoxyquinolin-4-yl)oxy)pyridin-2-yl)-1-propyl-4-(2,2,2-trifluoroethoxy)-1H-pyrazole-3-carboxamide ("Q702") was prepared, as described in WO 2019/229251.

This was used to identify stable, crystalline salts for development as therapeutic drug form. The approach was to generate solids under a wide and diverse range of nucleation conditions, designed to mimic the process conditions and solvents used during development and formulation. To this end, solvent-based experiments were performed, in particular evaporation experiments, vapour stress experiments (ambient and 40 °C), slurry experiments (ambient and 40 °C). All solids from these experiments were analysed by XRPD and the resulting patterns/forms compared to that exhibited by the starting material. Where sufficient material was available, further analysis (for example by NMR or thermal gravimetric analysis (TGA)) was conducted on solids with novel XRPD patterns/forms. Furthermore, salts were tested in terms of their solubility, in various solvents as well as in aqueous solutions.

More specifically, the following screening methods were performed:

### Salt Screening methods

Experiments were carried out at a scale of ~30 mg with 1:1 stoichiometry (salt former: free acid).

### Evaporation

A solution of Q702 was prepared in each solvent and filtered through a 0.2 µm PTFE filter. The filtered solution was evaporated in a fume hood at ambient temperature in a vial covered with perforated aluminium foil. The resulting solids were analysed by XRPD.

### Slurry experiments

Sufficient Q702 salt (amorphous/disordered salts) was added to a given solvent until undissolved solids remained at the desired temperature (20 or 40 °C). The vial was sealed and the slurry was maintained at the selected temperature and agitated by magnetic stirring for 1-11 days. Solids were isolated by centrifugation and air dried prior to analysis by XRPD.

### Vapour stress

Approximately 30 mg of Q702 salt (amorphous/disordered salts) was added to a vial and placed unsealed inside a larger sealed vessel containing 1 mL of the selected solvent. After 1-6 days, the samples were removed and analysed by XRPD. Vapour stressing was carried out at ambient and 40 °C.

Furthermore, the following techniques for analysis were used:

### Techniques for analysis of salts produced

### X-ray Powder Diffraction (XRPD)

XRPD analyses were performed using a Panalytical Xpert Pro diffractometer equipped with a Cu X-ray tube and a Pixcel detector system. The isothermal samples were analysed in transmission mode and held between low density polyethylene films. A default XRPD program was used (range 3-40°2θ, step size 0.013°, counting time 22 sec, ~5 min run time as well as a longer runs at (counting time 46 sec, ~11 min run time), (counting time 97 sec, ~22 min run time) and (counting time 591 sec, ~2 hour run time). XRPD forms were sorted, manipulated and indexed using HighScore Plus 2.2c software.

### Differential Scanning Calorimetry (DSC)

DSC analyses were carried out on a Perkin Elmer Jade Differential Scanning Calorimeter. Accurately weighed samples were placed in crimped aluminium pans (i.e. closed but not gas tight). Each sample was heated under nitrogen at a rate of 10 °C/minute to a maximum of 300 °C. Indium metal was used as the calibration standard. Temperatures were reported at the transition onset to the nearest 0.01 degree.

### Thermogravimetric Differential Thermal Analysis (TG/DTA)

Thermogravimetric analyses were carried out on a Mettler Toledo TGA/DSC1 STARe. The calibration standards were indium and tin. Samples were placed in an aluminium sample pan, inserted into the TG furnace and accurately weighed. The heat flow signal was stabilised for one minute at 30 °C, prior to heating to 300 °C in a stream of nitrogen at a rate of 10 °C/minute.

### Dynamic Vapour Sorption (DVS)

Dynamic Vapour Sorption (DVS) was performed using a Hiden Analytical Instruments IGAsorp Vapour Sorption Balance. Approximately 30 mg of sample was placed into a wire-mesh vapour sorption balance pan, loaded into the IGAsorp vapour sorption balance and held at 25 °C ± 0.1 °C. The sample was subjected to a step profile from 0 to 90%RH at 10% increments, followed by desorption from 90%RH to 0%RH at 10% increments. The equilibrium criterion was set to 99.0% step completion within a minimum of 60 minutes and a maximum of 5 hours for each increment. The weight change during the sorption cycle was monitored, allowing for the hygroscopic nature of the sample to be determined. The data collection interval was in seconds.

### ¹H / ¹³C Nuclear Magnetic Resonance spectroscopy (NMR)

NMR analysis was carried out on a Bruker 500MHz instrument in methanol-d₄ or DMSO-d₆. Instrumental parameters are listed on the relevant spectrum plots.

### Solubility measurements

Solubility was measured by a) solubility estimation by aliquot addition and b) HPLC:
a) Solubility estimation by aliquot-addition

Aliquots of the test solvent were added to an accurately weighed sample (~20 mg) of Q702 material, e.g. a particular Q702 salt, such as the isethionate salt of Q702 or the hydrochloride salt, at ambient temperature. The aliquot volumes were typically 100-1000 µL. Complete dissolution of the test material was determined by visual inspection. The solubility was estimated from these experiments based on the total solvent used to provide complete dissolution.

If dissolution did not occur after the last aliquot of solvent was added (typically ~50 volumes of solvent), the sample was subjected to two cycles of the following temperature cycling regime on the Clarity crystallization station:
- Heat from 20 °C to within 3 °C of solvent boiling point (or 100 °C, whichever was lower) at 0.5 °C/minute.
- Cool to 20 °C at 0.2 °C/minute.
- Stirrer speed 600 rpm.

From the infrared (IR) transmission data of the sample vials, dissolution and precipitation events were recorded as the point of complete transmission of IR and the onset of turbidity by IR respectively.

Samples were held at ambient temperature for 18 hours to maximize the chance of precipitation. Any recoverable solids were analyzed by XRPD. The solubility values for Q702 material were expressed as a range and rounded to the nearest whole number.

### b) HPLC for determination of equilibrium solubility

The HPLC method used to determine equilibrium solubility in a variety of solvents is outlined. The retention time of Q702 was typically 18.4 ± 0.143 min. A phenomenex Kinetex column was used with a particle size of 2.6 µm.

### Results

### Solubility of different Q702 salts in water/aqueous solutions

In terms of solubility, the various salts produced showed the following solubility values in aqueous solution, as summarized in the table 1:

**Table 1: Aqueous solubilities of various crystalline salts of Q702**

| **No.** | **Salt** | **Aq Solub (mg/mL)** | **pH of solution¹** | **XRPD² (see also following examples)** |
|---|---|---|---|---|
| 1 | Isethionate³ | 5.18 ± 0.113 | 3.32 | Insufficient material |
| 2 | Hydrochloride | 1.95 ± 0.01 | 2.68 | HCl form A |
| 3 | Ketoglutarate | 1.93 ± 0.04 | 2.91 | Insufficient material |
| 4 | Esylate³ | 1.23 ± 0.02 | 2.97 | Insufficient material |
| 5 | Phosphate | 1.04 ± 1.21 | 2.53 | disordered |
| 6 | Citrate | 0.85 ± 0.002 | 3.11 | New Form (Citrate B) |
| 7 | Edisylate | 0.83 ± 0.0005 | 2.31 | Changed to ESDA B |
| 8 | Oxalate | 0.46 ± 0.006 | 3.04 | New Form (disordered Oxalate B) |
| 9 | Malonate³ | 0.45 ± 0.03 | 3.38 | Malonate A |
| 10 | Sulfate | 0.27 ± 0.0001 | 2.06 | Changed to Sulfate B |
| 11 | Napsylate | 0.162 ± 0.0002 | 3.58 | Mixture of NSA A and B-disordered |
| 12 | Gentisic | 0.062 ± 0.00003 | 3 | Gentisic A |
| 13 | Tosylate | 0.026 ± 0.0004 | 4.26 | New Form (Tosylate B) |

| | | | | |
|---|---|---|---|---|
| 1. Crystalline salts slurried overnight in deionised water (pH = 6.8) and solution pH measured. 2. Crystalline salts slurried overnight in deionised water (pH = 6.8) and solids filtered off and analysed by XRPD 3. Solutions crashed out after being filtered and were re-dissolved in DMSO and ACN. Precipitation may be due to formation of less soluble salt form eg hydrate. | | | | |

As can be seen from the table 1, it turns out that the isethionate salt showed the highest solubility in aqueous solutions, however, it was not possible to reproduce such salt, especially not on a scale suitable for XRPD analysis, let alone for manufacturing purposes. The same applies to the ketoglutarate and the ethane sulfonate ("esylate") salt. The phosphate, citrate, ethane disulfonate and oxalate salts have substantially diminished aqueous solubility, and sulfate and tosylate salts are amongst the least aqueously soluble salts, whereas the hydrochloride salt shows a solubility of 1.95 mg/mL that is by far the best of all produced salts. Exactly why this is so remains to be elucidated. The fact that the hydrochloride salt is so much better than for example the sulfate (0.27 mg/mL) and the tosylate (0.026 mg/mL) salt which has the worst aqueous solubility is entirely surprising and was not to be expected from other systems, e.g. albendazole, where HCl, sulfate and tosylate salts have aqueous solubility values that are rather similar (Paulekuhn eet al, 2013, Pharmazie, volume 68, pages 555-5).

### Example 2

### Polymorph screen of Q702 HCl salt

Based on the surprising finding of Example 1, the present inventors then further studied the hydrochloric acid salt of Q702 to understand whether there were different polymorphic forms and to identify a stable form with suitable properties. To this end, crystallization experiments were performed, and the solids resulting therefrom were analysed by XRPD using patterns/forms compared to that exhibited by the starting material. To identify different forms of HCl-salts of Q702, the following screening methods were performed:

### Screening methods

### Temperature cycling

The test solvent (1 mL) was added to a sample of Q702 HCl salt (~20 mg) at ambient temperature and 2 cycles of the following temperature program was performed using the Clarity crystallisation station:
- Heat from 20 °C to 50-80 °C at 1 °C/min (depending on boiling point of solvent)
- Cool to 20 °C at 1 °C/min
- Stirrer speed - 600 rpm

### Slow evaporation

A solution of Q702 HCl salt was prepared in each solvent and filtered through a 0.2 µm PTFE filter. The filtered solution was evaporated in a fume hood at ambient temperature in a vial covered with perforated aluminium foil. The resulting solids were analysed by XRPD.

### Slurry experiments

Sufficient Q702 HCl salt (crystalline and/or amorphous) was added to a given solvent until undissolved solids remained at the desired temperature (5, 20, 40 or 50 °C). The vial was sealed and the slurry was maintained at the selected temperature and agitated by magnetic stirring for 5-8 days. Solids were isolated by centrifugation and dried under in air prior to analysis by XRPD.

### Vapour diffusion

A solution of Q702 HCl salt was filtered through a 0.2 µm PTFE filter into a clean vial. The vial was placed unsealed inside larger vials, which contained an aliquot of antisolvent. The larger vials were sealed and left undisturbed under ambient conditions. Samples did not form solids and therefore they were evaporated in air prior to analysis by XRPD.

### Sonication of pastes

Q702 HCl salt (~20 mg) was added to a vial with 50-150 µL of the selected solvent to form a paste. The mixture was sonicated at 70% intensity using a Cole-Parmer 130W ultrasonic processor using a pulsed program. In cases where the solids dissolved at ambient temperature, the sample was left uncapped to evaporate. The wet pastes recovered from these experiments were analysed using XRPD.

### Vapour stress

Approximately 20 mg of (crystalline and/or amorphous) was added to a vial and placed unsealed inside a larger sealed vessel containing 1 mL of the selected solvent. After 7-8 days, the samples were removed and analysed by XRPD.

### Humidity stress using crystalline material

Approximately 25 mg of Q702 HCl salt was added to four individual vials and placed unsealed into the following relative humidity chambers (sealed cabinets with relative humidity conditions controlled by super-saturated salt solutions) for 8 days prior to analysis by XRPD:
- Chamber 1 - 23% Relative Humidity
- Chamber 2 - 59% Relative Humidity
- Chamber 3 - 75% Relative Humidity
- Chamber 4 - 98% Relative Humidity

### Humidity stress using amorphous material

Approximately 20mg of amorphous Q702 HCl salt was added to two individual vials and placed unsealed into the following relative humidity chambers (sealed cabinets with relative humidity conditions controlled by super-saturated salt solutions) for 6 days prior to analysis by XRPD:
- Chamber 1 - 59% Relative Humidity
- Chamber 2 - 75% Relative Humidity at 40 °C

### Thermal stressing

Approximately 20mg of amorphous Q702 HCl salt was added to a vial, flushed with nitrogen, sealed and placed into a heater block at 50 °C for 7 days prior to analysis.

### Compression

Q702 HCl salt (50 mg) was added to a KBr pellet die and compressed overnight at ~740 MPa using a hydraulic press. The resultant solid disc was removed from the press and immediately analysed by XRPD.

### Milling

Approximately 50 mg of Q702 HCl salt was added to a milling chamber with an agate milling ball. Using a Retsch MM200 mixer mill, the material was milled for 3 x 2 minutes at a frequency of 25Hz. Periodically, the milling was stopped and powder that adhered to the milling chamber was scraped down. The resultant milled material was analysed using XRPD.

### Desolvation experiments

Samples were heated to ~100-116 °C on a hotplate and held for 20-40 min under a flow of nitrogen. Samples were cooled to ambient and analysed immediately by XRPD.

### Techniques for analysis of salts produced

For analysis of the different forms, various analysis techniques were used including X-ray Powder Diffraction (XRPD), Differential Scanning Calorimetry (DSC), Thermogravimetric Differential Thermal Analysis (TG/DTA), ¹H / ¹³C Nuclear Magnetic Resonance spectroscopy (NMR), Dynamic Vapour Sorption (DVS), solubility estimation by aliquot addition and equilibrium solubility measurements by HPLC, all as described in Example 1.

Moreover, the following additional analysis techniques were used:

### Volumetric Karl Fischer (KF) analysis for water content

Volumetric KF analysis was performed using a Mettler Toledo V30 KF titrator. A weighed amount of solvent was added to the KF cell via syringe. The solution was stirred and the water content of the sample was then determined by automatic titration against standard KF reagent titrant.

### Results

### 1. Solubility of Q702 HCl salt in different solvents

In terms of solubility in different solvents, the solubility of Q702 HCl salt was estimated in 9 solvent systems using the aliquot addition method. The Q702 HCl salt had a solubility of > 20 mg/mL in four of the solvents at ambient temperature. The solubility data obtained are shown in table 2. From these data and the solubility screen, the solvents were sorted into three groups outlined in the subsequent table to define the scope of the screening experiments.

**Table 2: Solubility estimates of Q702 HCl salt at 20 °C**

| **Solvent** | **Acronym** | **Solubility Range (mg/mL)** |
|---|---|---|
| Acetone | - | < 20 |
| Acetonitrile | ACN | < 20 |
| Dichloromethane | DCM | 66 - 100 |
| Dimethyl sulfoxide | DMSO | > 201 |
| Ethanol | EtOH | 67 - 101 |
| Ethyl acetate | EtOAc | < 20 |
| Methanol | MeOH | 49 - 66 |
| Methyl tert-butyl ether | MTBE | < 20 |
| Tetrahydrofuran | THF | < 20 |

**Table 3: Solvent systems grouped into categories**

| **(A) Solvents** | **(B) - Soluble with heating** | **(C) Anti-solvents** |
|---|---|---|
| Dichloromethane | Acetone | Ethyl acetate |
| Dimethyl sulfoxide | Acetonitrile | Methyl tert-butyl ether |
| Ethanol | Tetrahydrofuran | - |
| Methanol | - | - |

### 2. Polymorph screening

The present inventors tried to generate solids under a wide and diverse range of nucleation conditions, designed to mimic the process conditions and solvents used during development and formulation.

All solids from the crystallization experiments were analyzed by XRPD and the resulting patterns/forms compared to that exhibited by the starting material. Novel XRPD patterns/forms were assigned an alphabetical descriptor in order of discovery (Form B, Form C etc). Where sufficient material was available, further analysis (e.g. NMR or TGA) was conducted on solids with novel XRPD patterns/forms to allow tentative assignment of the novel pattern/form as a polymorph, solvate, hydrate, degradant or mixture thereof.

### Generation of amorphous material

A key objective of the experimental program was to obtain amorphous material for screening, as the present inventors believe such solids to have no 'memory' and subsequent stressing maximizes the chances of discovering novel crystal forms. In cases where amorphous material is very unstable at ambient temperature, disordered (poor crystallinity) material can be used instead.

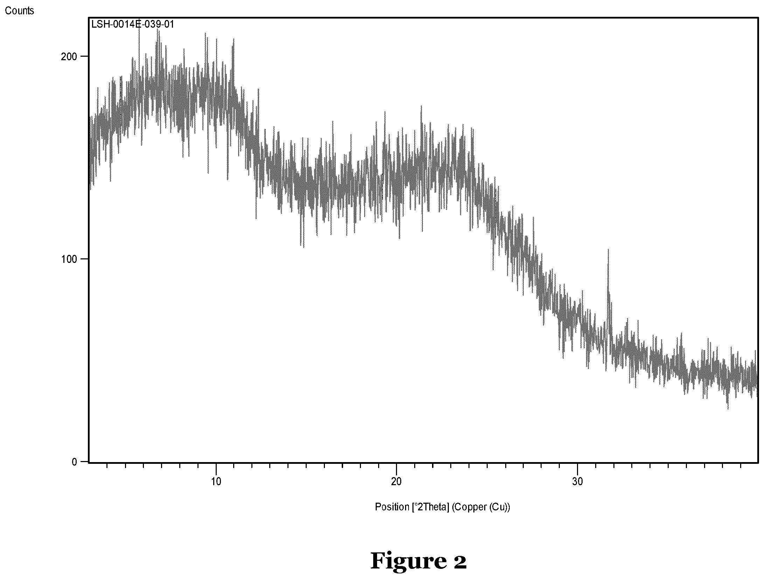

Amorphous material was isolated by freeze drying. A solution was prepared of Q702 free base material (7.5 g batch) in a 50:50 mixture of acetonitrile (ACN):water with 0.5M HCl acid before freezing in liquid nitrogen and drying under vacuum. A white material was obtained which was shown to be amorphous by XRPD (Figure 2). The ¹H NMR spectrum of the material (data not shown) conformed to the molecular structure and no solvent was detected.

### Solvent based screening techniques

Solvent based experiments were performed on approximately 20 mg scale in glass vials. The methods employed are described in detail further above. The present inventors believe that varying the nucleation conditions in this way maximizes the chance of finding new forms and also the frequency of occurrence of these forms under typical processing conditions. Different patterns/forms emerged from different experiments and were classified into 18 different forms A - R according to the analysis obtained by XRPD.

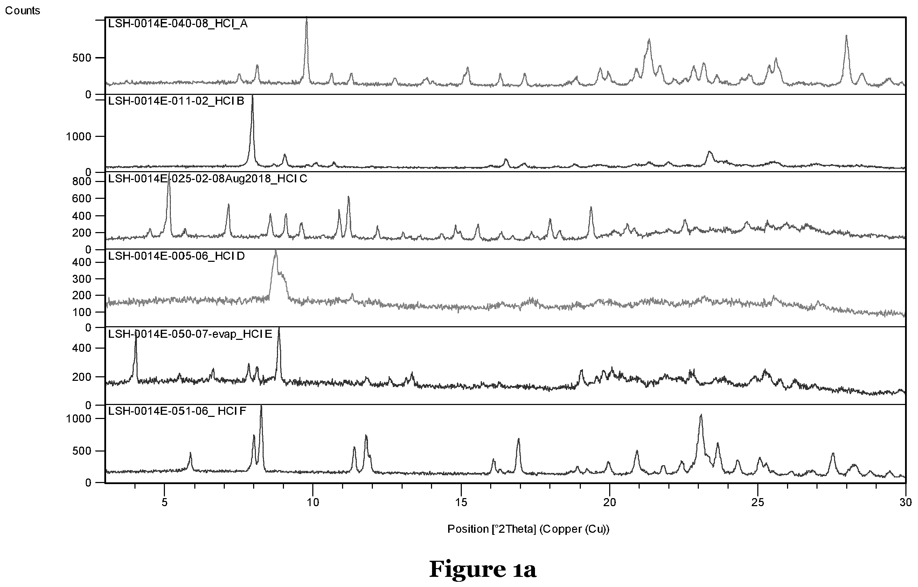

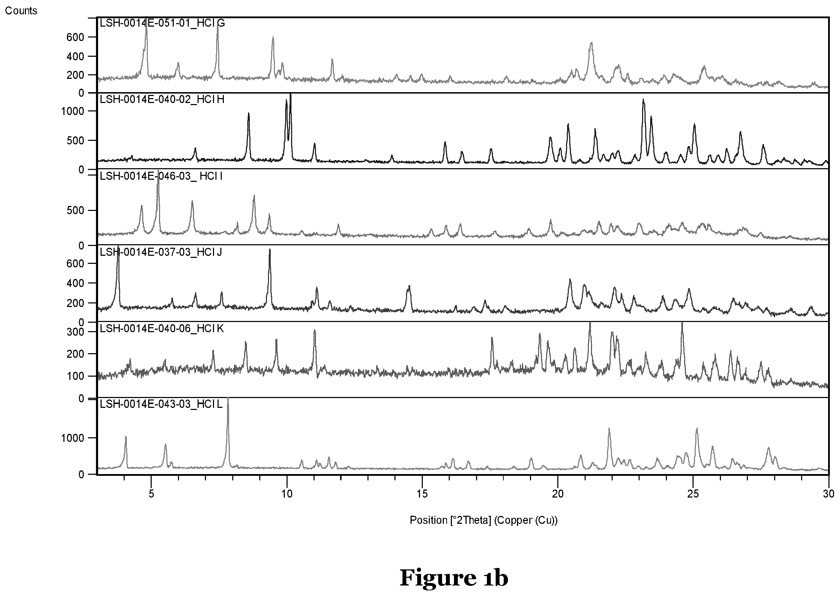

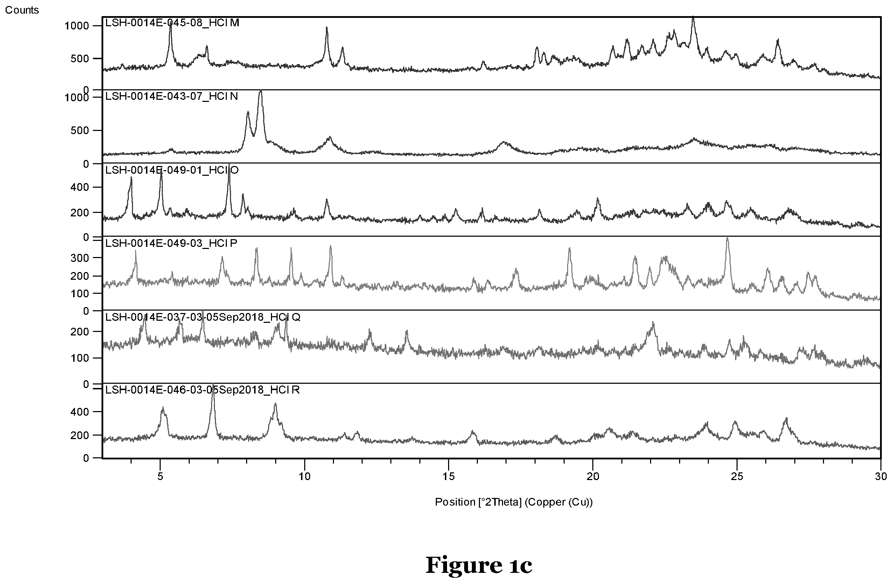

Altogether, eighteen unique crystalline solids (summarized in the table 4) were obtained during this study and labelled forms A-R, based on their XRPD patterns/forms shown in figures 1a - 1c. Amorphous material was also generated from freeze drying of Q702 free base in ACN:water with 0.5M HCl.

**Table 4: Summary of the physical forms obtained by the inventors**

| Form | Comment |
|---|---|
| A | Crystalline, hydrate |
| B | Crystalline, hydrate |
| C | Crystalline, likely a hydrate |
| D | Disordered solid |
| E | Crystalline, hydrate or anhydrate |
| F | Crystalline, solvate |
| G | Crystalline, solvate |
| H | Crystalline, likely a ACN solvate |
| I | Crystalline, likely a EtOH solvate |
| J | Crystalline, possible Dioxane solvate |
| K | Crystalline, DMSO solvate |
| L | Crystalline, possible MeOH solvate |
| M | Disordered, Dioxane solvate |
| N | Disordered solid, possible hydrate or solvate |
| O | Crystalline, likely a DMSO solvate |
| P | Crystalline, possible DMSO solvate |
| Q | Disordered solid, possible hydrate or anhydrate |
| R | Disordered solid, possible hydrate or anhydrate |
| Amorphous | Prepared by freeze drying in ACN:water |

More specifically, in the generation of these various forms, the following screening techniques were employed:

### Temperature cycling

Samples were subjected to the temperature cycling program outlined further above and the results are shown in the table 5. Three novel solids designated, forms F, G and H were obtained. HCl form F was isolated from EtOAc, form G was isolated from acetone and form H was isolated as a mixture from ACN.

**Table 5: Screening results from temperature cycling experiments**

| **Input (LSH-0014E-)** | **Sample No. (LSH-0014E-)** | **Solvent** | **Antisolvent** | **XRPD** |
|---|---|---|---|---|
| 034-02_1 (Form C) | 051-01 | Acetone | none | G |
| 034-02_1 (Form C) | 051-02 | ACN | none | H+ unknown |
| 034-02_1 (Form C) | 051-06 | EtOAc | none | F |
| 034-02_1 (Form C) | 051-08 | MTBE | none | disordered C |
| 034-02_1 (Form C) | 051-09 | THF | none | disordered C |

### Evaporation in vials

Slow evaporation of Q702 HCl salt solutions were conducted as described further above. The results are shown in the table 6. Two novel solids designated, form E and form G were obtained. Form E was isolated twice using both EtOH and MeOH, while, form G with additional peaks at 7.9 and 8.9°2theta was isolated using acetone. Amorphous material was obtained from DMSO while a mixture of amorphous and HCl form C material were obtained from DCM.

For vapor diffusion experiments at ambient temperature which did not produce any solid material were evaporated in air for a few days (see table 7). Two novel solids designated, form L and form P were obtained. Disordered form L was isolated from an experiment in EtOH using MTBE as the antisolvent while form P was isolated from an experiment in DMSO using MTBE as the antisolvent.

For vapor diffusion experiments at 40 °C which did not produce any solid material they were evaporated in air for a few days (see table "8). One experiment in EtOH using water as the antisolvent generated form B material minus a peak at 9.8°2theta. One novel solid termed, form O was isolated from DMSO using water as the antisolvent. An experiment in MeOH using water as the antisolvent afforded disordered form C with an additional peak at 8.0°2theta.

**Table 6: Screening results from evaporations in vials**

| **Input (LSH-0014E-)** | **Sample No. (LSH-0014E-)** | **Solvent** | **Result** | **XRPD** |
|---|---|---|---|---|
| 034-02_1 (Form C) | 050-03 | DCM | solid | amorphous + C |
| 034-02_1 (Form C) | 050-04 | DMSO | solid | amorphous |
| 034-02_1 (Form C) | 050-05 | Ethanol | solid | disordered E |
| 034-02_1 (Form C) | 050-07 | MeOH | solid | E |
| amorphous | 052-01 | Acetone | solid | G + peaks at 7.9 and 8.9°2theta |

**Table 7: Results from evaporation of vapor diffusion experiments at ambient temperature**

| **Input (LSH-0014E-)** | **Sample No. (LSH-0014E-)** | **Solvent** | **Antisolvent** | **XRPD** |
|---|---|---|---|---|
| 034-02_1 (Form C) | 049-03 | DMSO | MTBE | P |
| 034-02_1 (Form C) | 049-04 | Ethanol | MTBE | disordered L |

**Table 8: Results from evaporation of vapor diffusion experiments at 40 °C**

| **Input (LSH-0014E-)** | **Sample No. (LSH-0014E-)** | **Solvent** | **Antisolvent** | **XRPD** |
|---|---|---|---|---|
| 034-02_1 (Form C) | 049-01 | DMSO | water | O |
| 034-02_1 (Form C) | 049-02 | Ethanol | water | B (minus peak at 9.8°2theta) |
| 034-02_1 (Form C) | 049-05 | MeOH | water | disordered C + peak at 8.0°2theta |

### Slurry experiments at 5 °C

Suspensions of amorphous Q702 HCl salt in various solvents were slurried at 5 °C for 7-8 days prior to isolation and analysis by XRPD (see table 9). Two experiments, one in MTBE and another in a 5:45 mixture of DCM:Heptane yielded amorphous material.

Another three experiments in a 5:45 mixture of Ethanol:water, a 5:45 mixture of Acetone:water and a 5;45 mixture of THF:water contained form B material with additional peaks. Form G material was obtained in an experiment in acetone while form F was isolated three times in EtOAc, MEK (Methyl ethyl ketone) and iPrOAc (Isopropyl acetate).

**Table 9: Screening results from slurry experiments at 5 °C**

| **Sample No. (LH-0014E-)** | **Solvent** | **XRPD** |
|---|---|---|
| 042-01 | Acetone | G |
| 042-02 | MTBE | amorphous |
| 042-03 | (5:45) Ethanol:water | B+ peak at 5.1°2theta |
| 042-04 | EtOAc | F |
| 042-05 | (50:50) MeOH:ACN | E |
| 042-06 | (5:45) DCM:Heptane | amorphous |
| 042-07 | (5:45) Acetone:water | B+ peak at 5.1 and 22.5°2theta |
| 042-08 | (5:45) THF:water | B |
| 042-09 | (50:50) Ethanol:MTBE | E |
| 042-10 | MEK | F |
| 042-11 | iPrOAc | F |

### Slurry experiments at 20 °C

Suspensions of Q702 free base material in various concentrations of acid stock solutions were stirred at 20 °C for 5 days prior to isolation and analysis by XRPD (see table 10).

**Table 10: Screening results from slurry experiments at 20 °C**

| **Sample No. (LH-0014E-)** | **Sample No. (LH-0014E-)** | **Solvent** | **Acid solution** | **XRPD** |
|---|---|---|---|---|
| as-received FB | 037-01 | dioxane | 4 M HCl in dioxane | J |
| as-received FB | 037-02 | water | 1 M HCl in water | B+C |
| as-received FB | 037-03 | dioxane | 4 M HCl in dioxane | J |
| as-received FB | 037-04 | water | 1 M HCl in water | disordered C + peak at 8.0°2theta |
| as-received FB | 037-06 | MeOH | 1 M HCl in water | L |

| | | | | |
|---|---|---|---|---|
| FB: Free base | | | | |

### Slurry experiments at 40 °C and 50 °C

Suspensions of Q702 HCl salt in various solvents were stirred at 50 °C for 6 days prior to isolation and analysis by XRPD (see table 11). One novel solid designated, form K was isolated from a 1:3 mixture of DMSO:MTBE. Form A was isolated from two experiments a 1:3 mixture of EtOH:water and a 1:3 mixture of MeOH:water. Form C was obtained from two experiments in MTBE and MIBK, while form H was isolated from ACN. One experiment in acetone at 40 °C obtained disordered form G material.

**Table 11: Screening results from slurry experiments at 40 °C and 50 °C**

| **Sample No. (LH-0014E-)** | **Solvent** | **Temp (°C)** | **XRPD** |
|---|---|---|---|
| 040-01 | acetone | 40 | disordered G |
| 040-02 | ACN | 50 | H |
| 040-03 | (1:3) Ethanol:water | 50 | A |
| 040-04 | EtOAc | 50 | F |
| 040-05 | MTBE | 50 | C |
| 040-06 | (1:3) DMSO:MTBE | 50 | K |
| 040-07 | THF | 50 | disordered C + A |
| 040-08 | (1:3) MeOH:water | 50 | A |
| 040-09 | MIBK | 50 | C |

### Vapour diffusion

Vapour diffusion experiments were carried out as described further above. However, neither experiment yielded any solid material.

### Vapor diffusion at 40 °C

Vapor diffusion experiments at 40 °C were carried out as described further above. However, all experiments did not yield any solid material.

### Sonication

Suspensions of amorphous Q702 HCl salt were prepared in various solvents and subjected to a pulsed program as outlined above. Samples were stored at 5 °C prior to isolation and analysis by XRPD (see table 12). For two experiments, one in a 20:80 mixture of Acetone:MTBE and one in DCM, the sample remained as amorphous material. Disordered form C, F and H were isolated form iPrOAc, ACN and EtOAc. One experiment in THF obtained form C material. Two novel solids designated form I and form M was obtained. Form I with amorphous content was isolated from ethanol while form M was isolated from dioxane.

**Table 12: Screening results from sonication experiments**

| **Sample No. (LH-0012E-)** | **Solvent** | **XRPD** | **Cycles** |
|---|---|---|---|
| 045-01 | Ethanol¹ | I + amorphous | 8 |
| 045-02 | EtOAc | disordered F | 5 |
| 045-03 | ACN | disordered H | 8 |
| 045-04 | (20:80) Acetone:MTBE | amorphous | 6 |
| 045-05 | THF¹ | C | 8 |
| 045-06 | DCM | amorphous | 3 |
| 045-07 | iPrOAc | disordered C | 8 |
| 045-08 | Dioxane | M | 8 |

| | | | |
|---|---|---|---|
| 1. This experiment went into solution before being refrigerated. The solid which formed was then analyzed by XRPD. | | | |

### Solid state screening techniques

The non-solvent based (solid state) screening methods include ball milling, compression thermal, vapor and humidity stressing (see further above). These techniques mimic conditions that are likely to be encountered in large scale processing, e.g. on hot reactor walls or during drying and tableting operations. The present inventors believe that varying the nucleation conditions in this way maximizes the chance of finding new forms and also the frequency of occurrence of these forms under typical processing conditions.

### Compression

A die press was used to mimic the uniaxial stress experienced during tableting, which can reach up to 300 MPa. Samples were analyzed soon after decompression in order to limit the likelihood of a form conversion at 1 bar pressure (see table 13). The diffractogram (not shown) shows that the sample is composed of disordered HCl C material.

**Table 13: Screening results from compression experiments**

| **Input (LSH-0014E-)** | **Sample No. (LH-0014E-)** | **Conditions** | **Result** | **XRPD** | **Observation** |
|---|---|---|---|---|---|
| 034-02_1 (Form C) | 048-01 | 740MPa, overnight | solid | disordered HCl C | off white solid |

### Milling

A ball mill was used to mimic the effects of grinding that would be experienced during formulation steps such as dry blending and wet granulation. Milling was performed on a sample of Q702 HCl salt. The sample was analyzed soon after completion of milling in order to limit the likelihood of a further change in form (see table 14). From the resultant XRPD (not shown) it becomes clear that when dry milled HCl form C material becomes more disordered.

**Table 14: Screening results from milling experiments**

| **Input (LSH-0014E-)** | **Sample No. (LH-0014E-)** | **Conditions** | **Result** | **XRPD** |
|---|---|---|---|---|
| 047-01 (Form C) | 034-03 | 3x2 min, 25Hz, dry | solid | disordered HCl C |

### Desolvation

Desolvation of solvated or hydrated compounds can be a useful method for screening for novel solid forms. Samples were heated to the desired temperature under a flow of nitrogen for 20-40 minutes. The results are shown in the following table. Q702 HCl form C material remained as form C, while Q702 HCl form A+C remained as form A+C.

**Table 15: Results from desolvations experiments**

| **Input (LSH-0014E-)** | **Sample No. (LH-0014E-)** | **Solvent** | **Temp (°C)** | **Result** | **XRPD** |
|---|---|---|---|---|---|
| 034-02_1 (form C) | 058-01 | acetone | 100 | solid | C |
| 059-01-Aliquot (form A+C) | 058-02 | MTBE | 116 | solid | A+C |

### Thermal stress

Amorphous Q702 HCl salt was thermally stressed at 50 °C for 7 days under nitrogen in a sealed vial and analyzed by XRPD. The sample as shown in the table 16 remained amorphous.

**Table 16: Screening results from thermal stress experiment**

| **Input** | **Sample No (LH-0012E-)** | **Conditions** | **Result** | **XRPD** | **Observation** |
|---|---|---|---|---|---|
| amorphous | 044-01 | 50 °C; 7 days | solid | amorphous | white solid |

### Vapor stress

X-ray amorphous material generated from freeze drying was exposed to air saturated in solvent vapor for 7-8 days before being analyzed by XRPD. As amorphous material has lost long range order, it is in a high energy state. Exposure to vapor plasticizes the solid, allowing limited molecular mobility and is therefore a useful method of generating metastable solvates and hydrates.

For the vapor stress experiments carried out using amorphous material two experiments obtained disordered form C material. Two experiments, one in a 20:80 mixture of EtOH:EtOAc and another in EtOAc yielded form F material. Form G material was isolated from MEK while disordered form G material was isolated from THF. One experiment in a 50:50 mixture of EtOH:MTBE yielded disordered form I+A material. form L material was obtained from a 50:50 mixture of MeOH:THF. One novel solid designated, form N was isolated from a 20:80 mixture of DMSO:water. The results are shown in the table 17.

Vapor stress experiments were also carried out using Q702 HCl salt at ambient temperature. One experiment was carried out using Q702 free base material. The results are shown in the table 18. Form I material was isolated from ethanol while disordered form H material was isolated from ACN. One experiment in a 13:1 mixture of THF:water yielded disordered form C material while an experiment in MTBE yielded form C with amorphous content. One experiment was carried out using Q702 free base material with 0.5M HCl in water. MeOH was used as the solvent and it yielded form L material.

**Table 17: Results from vapor stressing using amorphous Q702 material**

| **Sample No. (LSH-0014E-)** | **Solvent** | **XRPD** |
|---|---|---|
| 043-01 | (20:80) Acetone:MTBE | disordered C |
| 043-02 | (20:80) Ethanol:EtOAc | F |
| 043-03 | (50:50) MeOH:THF | L |
| 043-04 | (25:75) ACN:water | D |
| 043-05 | THF | disordered G |
| 043-06 | EtOAc | F |
| 043-07 | (20:80) DMSO:water | N |
| 043-08 | (50:50) Ethanol:MTBE | disordered I +A |
| 043-09 | MEK | G |
| 043-10 | iPrOAc | disordered C |

**Table 18: Screening results from vapor stress experiments using crystalline Q702 material**

| **Sample No. (LH-0014E-)** | **Sample No. (LH-0014E-)** | **Solvent** | **XRPD** | **Comments** |
|---|---|---|---|---|
| 034-02_1 (Form C) | 046-01 | THF/water (13:1, A_{w}~0.9) | disordered C | - |
| 034-02_1 (Form C) | 046-02 | ACN | disordered H | - |
| 034-02_1 (Form C) | 046-03 | Ethanol | I | - |
| 034-02_1 (Form C) | 046-04 | MTBE | amorphous + C | small sample very static |
| Q702 freebase | 037-05 | MeOH | L | 0.5M HCl in water |

### Humidity stress

X-ray amorphous material generated from freeze drying was exposed to various controlled humidity conditions for 6 days before analysis by XRPD. The results are shown in the table 19. Both samples remained as amorphous material. A weight gain of 6.13% was observed for the sample stressed at 40 °C/75%RH (see table 20), suggesting that the material is hygroscopic.

Humidity stress experiments were also carried out using crystalline Q702 HCl form C material and were stressed for 8 days. The results are shown in the table 21. All samples remained as Q702 HCl form C material. A weight gain of 2.99% was observed for the sample stressed at 98%RH (see table 22). A weight loss of 11.05% was observed for the sample stressed at 75%RH stress, while at 40°C/75%RH a weight loss of 1.96% was observed.

**Table 19: Results from humidity experiments using amorphous material**

| **Input material** | **Sample No. (LH-0014E-)** | **Screen method** | **Result** | **XRPD** |
|---|---|---|---|---|
| amorphous | 041-01 | 59%RH stress | solid | amorphous |
| amorphous | 041-02 | 40°C/75%RH stress | solid | amorphous |

**Table 20: Weight change of sample stressed using amorphous material at 40°C/75%RH**

| Sample No. (LH-0012E-) | Screen method | Initial weight (mg) | Final weight (mg) | Weight change (mg) | % Weight change |
|---|---|---|---|---|---|
| 041-02 | 40°C/75%RH stress | 21.76 | 23.1 | +1.34 | +6.13 |

**Table 21: Results from humidity experiments using HCl form C material**

| **Input material (LSH-0014E-)** | **Sample No. (LH-0014E-)** | **Screen method** | **Result** | **XRPD** |
|---|---|---|---|---|
| 034-03_29 (Form C) | 053-01 | 98%RH stress | solid | C |
| 034-03_29 (Form C) | 053-02 | 75%RH stress | solid | C |
| 034-03_29 (Form C) | 053-03 | 59%RH stress | solid | C |
| 034-03_29 (Form C) | 053-04 | 23%RH stress | solid | C |
| 034-03_29 (Form C) | 053-05 | 40°C/75%RH stress | solid | C |

**Table 22: Weight change of sample stressed using crystalline HCl form C material**

| Sample No. (LH-0012E-) | Screen method | Initial weight (mg) | Final weight (mg) | Weight change (mg) | % Weight change |
|---|---|---|---|---|---|
| 053-01 | 98%RH stress | 20.1 | 20.7 | +0.60 | +2.99 |
| 053-02 | 75%RH stress | 19.0 | 16.9 | -2.10 | -11.05 |
| 053-05 | 40°C/75%RH stress | 20.4 | 20.0 | -0.40 | -1.96 |

### Conclusions from polymorph screening

Approximately 80 experiments were carried out using solvent and non-solvent based techniques. Eighteen unique crystalline solids (see table 4) were observed during this study and labelled Forms A-R. Amorphous material was also generated from freeze drying of Q702 free base in ACN:water with 0.5M HCl.

Using the methodology as described here altogether, 18 different patterns/forms were identified and prepared, using different techniques as summarized in the table 23:

**Table 23: Preparation of different forms of HCl salts of Q702**

| **Material** | **Preparation Conditions** | **Comments** |
|---|---|---|
| HCl salt form A | Slurries from THF, MeOH or EtOH water mixtures | Crystalline, probable hydrate |
| HCl salt form B | Multiple methods from ACN, EtOH, acetone or THF water mixtures | Crystalline, hydrate |
| HCl salt form C | Slurry of HCl D in acetone-water | Crystalline hydrate (with some amorphous content) |
| HCl salt form D | Multiple methods in THF or ACN water mixtures. | Disordered solid |
| HCl salt form E | Slow Evap or 5°C slurry from MeOH or EtOH | Crystalline, anhydrate or hydrate |
| HCl salt form F | Multiple methods from EtOAc | Crystalline, EtOAc solvate |
| HCl salt form G | Multiple methods from acetone, THF or MEK | Crystalline, solvate |
| HCl salt form H | Multiple methods from ACN | Crystalline, likely a ACN solvate |
| HCl salt form I | VS or sonication in EtOH or EtOH-MTBE | Crystalline, likely a EtOH solvate |
| HCl salt form J | RT slurry in dioxane | Crystalline, likely a Dioxane solvate |
| HCl salt form K | 40°C slurry in DMSO-MTBE | Crystalline, DMSO solvate |
| HCl salt form L | Multiple methods form MeOH or EtOH | Crystalline, possible MeOH or EtOH solvate |
| HCl salt form M | Sonication in dioxane | Disordered, possible Dioxane solvate |
| HCl salt form N | VS in DMSO-water | Disordered solid possible hydrate or DMSO solvate. |
| HCl salt form O | Evap in DMSO-water | Crystalline, likely a DMSO solvate |
| HCl salt form P | Evap in DMSO-MTBE | Crystalline, possible DMSO solvate |
| HCl salt form Q | Pat I reanalysed after 7 days | Disordered solid, possible hydrate or anhydrate |
| HCl salt form R | Pat J reanalysed after 5 days | Disordered solid, possible hydrate or anhydrate |

| | | |
|---|---|---|
| Evap=evaporation, VS=vapour stress | | |

The amorphous form of the HCl salt of Q702 represents the starting point for various other crystalline forms. As it turns out, however, with the exception of forms A, B, C, D, E, Q and R, all the other forms produced in this example are solvates. Moreover, the non-solvate forms B, D, E, Q and R have a low crystallinity; therefore the two most promising crystalline patterns/forms were non-solvated form A and form C.

### Example 3

### Comparison of form A and C of Q702 HCl salt

### Preparation of form C of Q702 HCl salt

### Preparation of sample having form C

Q702 free base (~500 mg) was weighed into a glass vial. THF inhibitor free (9.38 mL) was added with 0.5M HCl (1.883 mL). The sample was evaporated under nitrogen to dry. It was seeded with HCl form C material once it began to crystallise. The material was left to dry in the air. Once dry it was analysed by XRPD: form B+C.

A 20:80 mixture of Acetone:water (3333 µL) was added to the sample and left to stir for two days at 40 °C. The sample was centrifuged for 3 mins and the liquid decanted. The sample was left to dry in air and then analysed by XRPD: HCl C material.

### Characterisation of Q702 HCl salt form C material

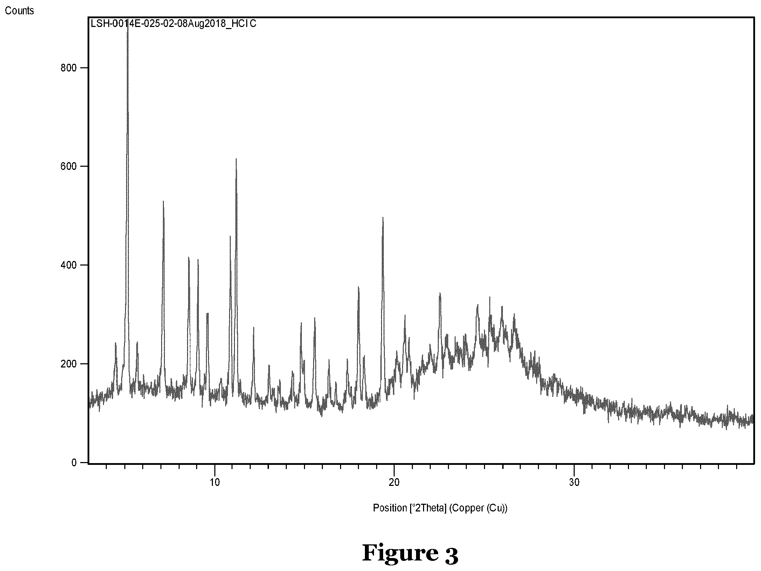

Q702 HCl salt Form C material was characterised by a variety of analytical techniques including XRPD, TGA, DSC, ¹H NMR, ¹³C NMR, PLM, DVS, FT-IR, UV-vis, IDR and CHN elemental analysis. XRPD data indicated that the material was crystalline but disordered and contained some amorphous content even after attempts to improve crystallinity (see figure 3).

Different batches of form C material have been analysed by TGA using open and/or closed pan configurations to better understand the nature of the material.

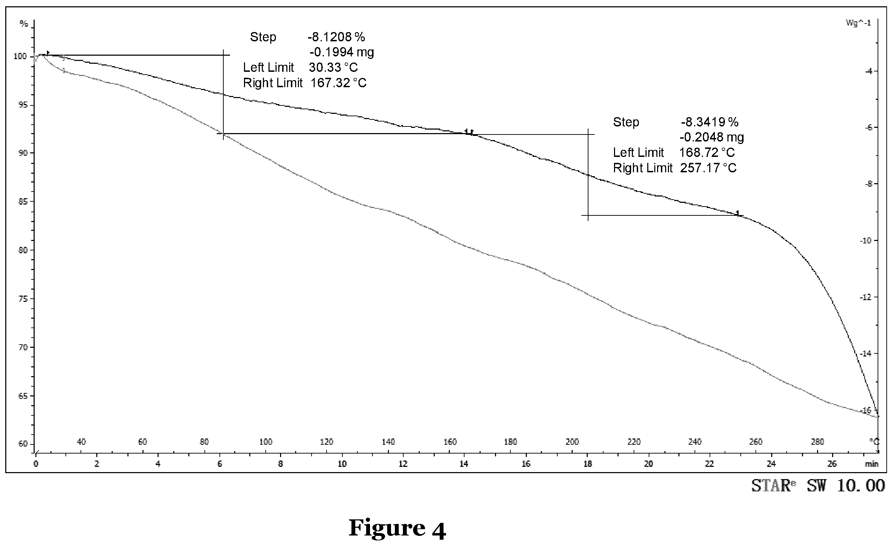

Using an open pan lid the sample lost 8% weight from 30-167 °C during TG analysis (see figure 4) followed by a further 8% weight loss from 167-257 °C. The combined weight loss equates to 6 mol eq water. HCl content of a 1:1 salt is 6.4% so weight loss is too large for loss of HCl. This suggests HCl salt form C material is likely hydrated. Another sample lost 5.7% weight from 30-166 °C and a further weight loss of 6.1% from 167-236 °C. A combined weight loss equates to 4 mol eq water. The difference in weight loss could be due to samples not being completely dry.

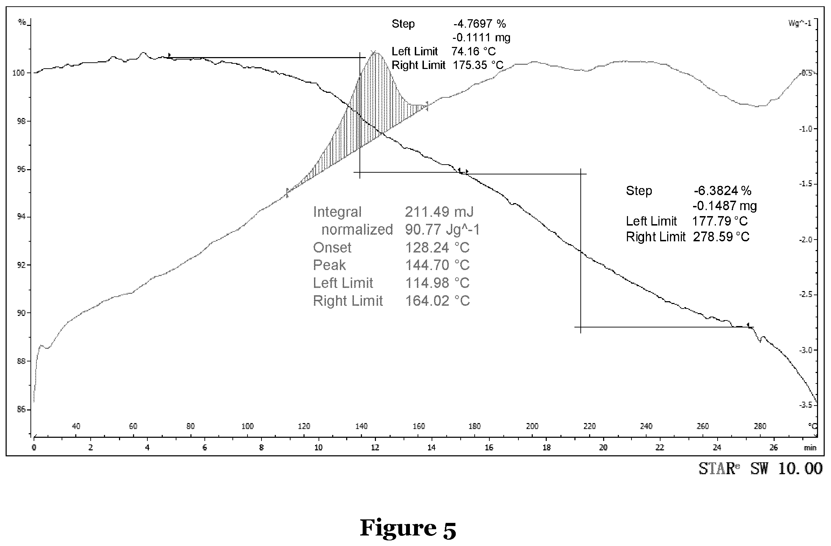

Much smaller weight loses were seen with closed pan configuration due to the pan lid slowing vaporisation. One sample (see figure 5) showed a weight loss of 4.8% from 74-175 °C and a further 6.3% weight loss from 178-279 °C. Another sample showed 3% weight loss from 30-90 °C during TG analysis followed by a 22% weight loss from 90-120 °C likely due to loss of water (up to 3 mol eq). However, this could be due to the sample being wet.

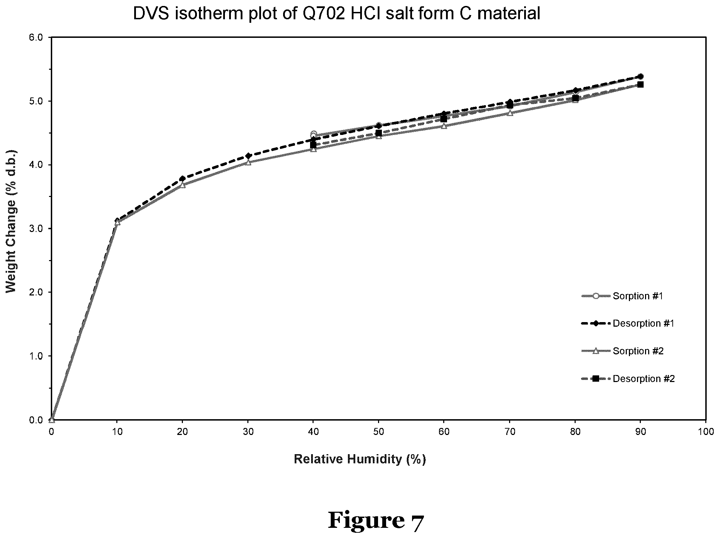

Hygroscopicity was assessed by DVS analysis (see figure 7). The isothermal plot showed the total weight gain between 40-80%RH was 0.7%w/w and indicates that the sample is slightly hygroscopic, based on the European Pharmacopoeia classification. XRPD analysis of post DVS sample (data not shown) matched that of HCl form C and no physical change in the sample was detected.

HCl form C was stressed at 40 °C/75% relative humidity for 15 days. XRPD analysis showed that it remained as HCl salt form C.¹H NMR spectrum indicated that the sample conformed to the molecular structure and no solvent was detected (data not shown).

HCl form C was desolvated in a hotplate under a flow of nitrogen at 100 °C. XRPD analysis showed the sample remained as HCl C material (data not shown).

### Preparation of the form A of the HCl salt of Q702

### Q702 HCl salt form A material

Form A material was generated from several different experiments as listed in the table 24 but two samples were a mixture containing also form C material. Form A material was crystalline by XRPD analysis (see figure 8).

**Table 24: Production of form A**

| **Input (LSH-0014E-)** | **Sample No. (LSH-0014E-)** | **Solvent** | **Screen method** | **Result** | **XRPD** |
|---|---|---|---|---|---|
| 034-02_1 (Form C) | 040-03 | Ethanol-water | slurry (50 °C) | solid | A |
| 034-02_1 (Form C) | 040-07 | THF | slurry (50 °C) | solid | disordered C + A peaks |
| 034-02_1 (Form C) | 040-08 | MeOH-water | slurry (50 °C) | solid | A |
| 059-01-evap (Form A+C) | 059-01-18sep2018 | (94:6) THF:water | slurry (20 °C) | solid | A+C |
| 059-01-18sep2018 (Form A+C) | 059-01-19sep2018 | (94:6) THF:water | slurry (40 °C) | solid | A |
| 059-01-19sep2018 (Form A) | 060-01-15 day stress | none | 40 °C/75% RH | solid | A |

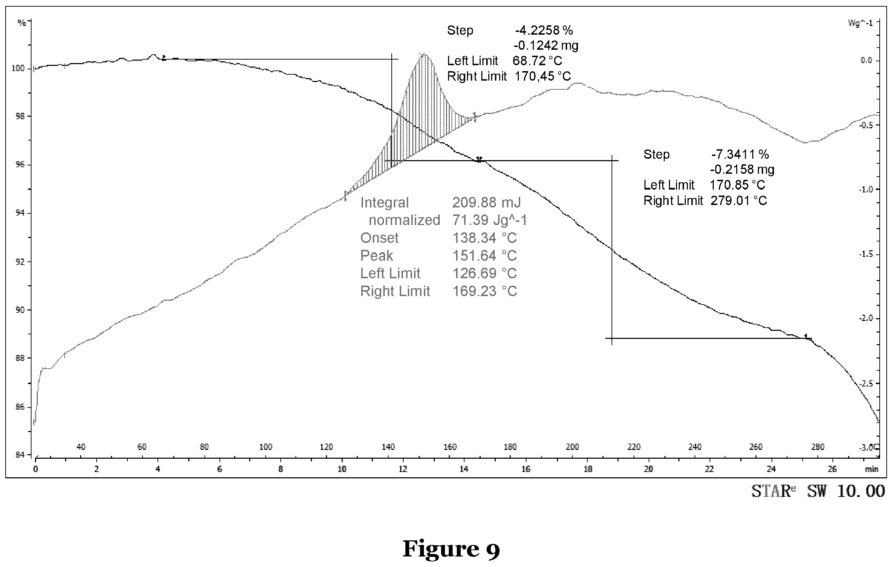

TG analysis of the HCl form A using a closed pan showed a continual gradual weight loss from 60 °C (see figure 9). The large endotherm is likely due to the melt, with onset temperature of 139 °C. A weight loss of 4.2 % was noted between 69-171 °C and equates to 1.4 mol eq of water. A second weight loss of 7.3% was noted from 171-279 °C.

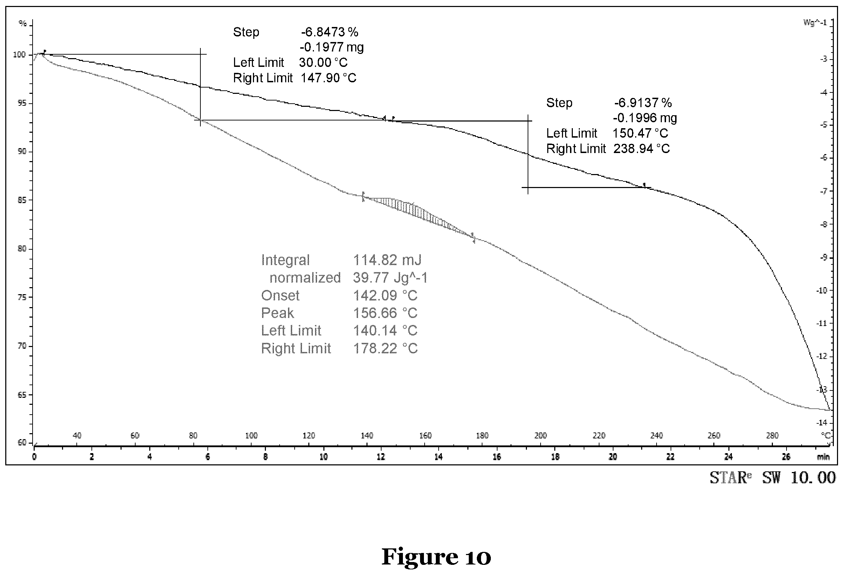

TG/DSC analysis of the HCl form A using an open pan showed a weight loss of 6.8% from 30-145 °C which equates to 2 mol eq water (see figure 10). A second weight loss of 6.9% was noted from 151-239 °C which is likely due to the melt. Onset was observed at 142 °C.

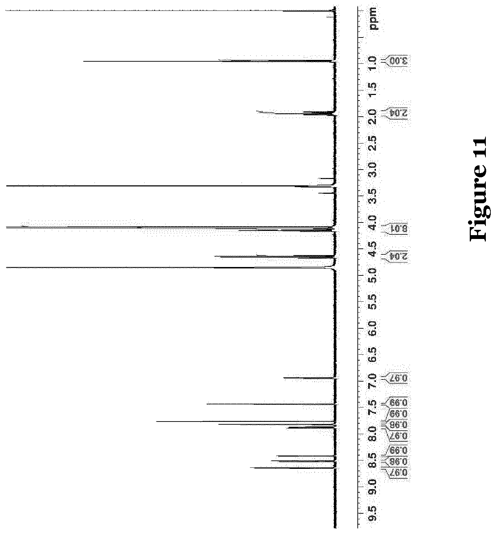

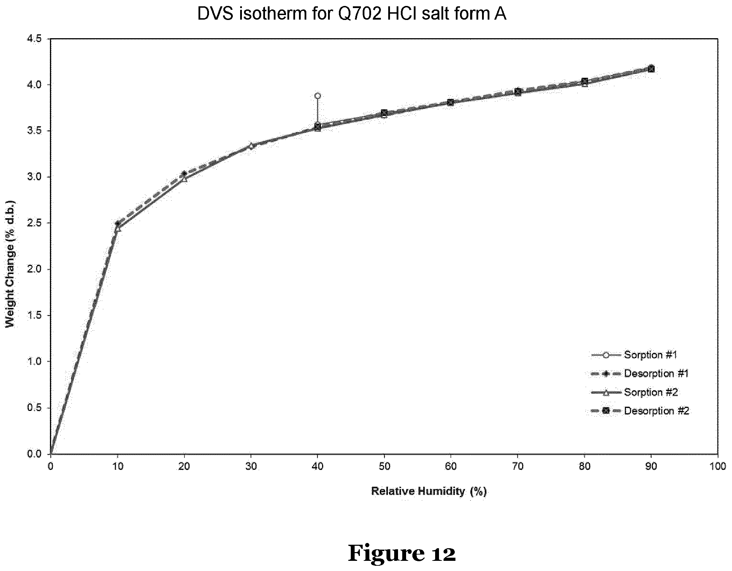

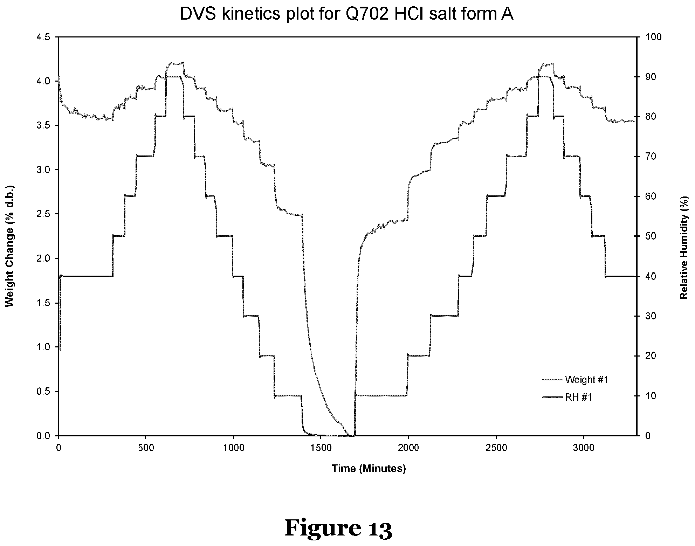

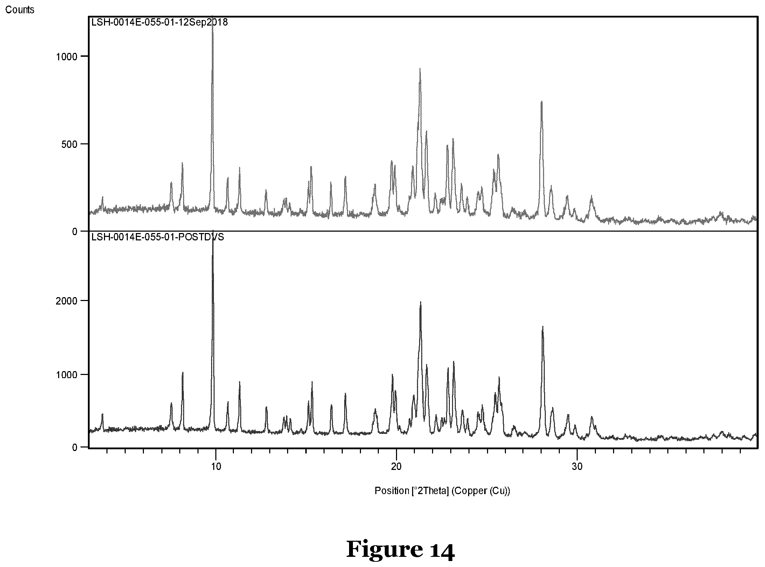

The ¹H NMR spectrum for form A material indicated that the sample conformed to the molecular structure (see figure 11). Solvent was not detected. Peak shifting was observed compared with spectrum of Q702 free base which suggests salt formation has occurred. Hygroscopicity was assessed by DVS analysis (see figures 12 and 13). The isothermal plot showed the total weight gain between 40-80%RH was 0.5%w/w and indicates that the sample is slightly hygroscopic, based on the European Pharmacopoeia classification. XRPD analysis of post DVS sample (see figure 14) matched that of the HCl form A and no physical change in the sample was detected.

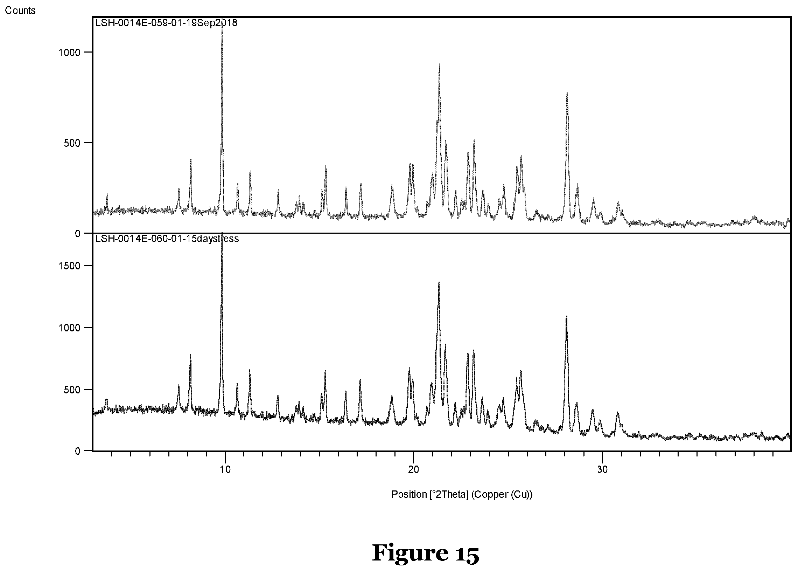

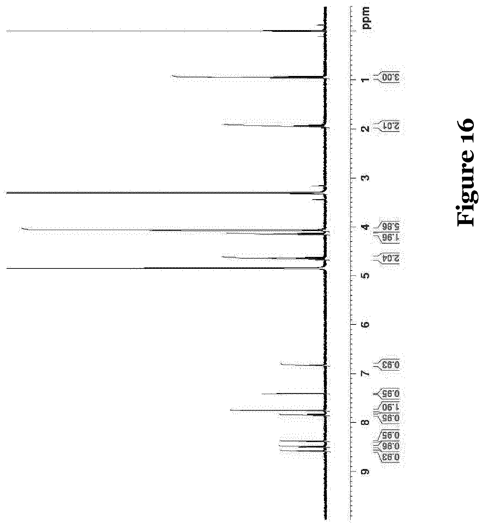

Form A was stressed at 40 °C/75% for 15 days. Post stress XRPD analysis showed the material remained as form A (see figure 15). The ¹H NMR spectrum for form A material indicated that the sample conformed to the molecular structure (see figure 16). Solvent was not detected. Hence, it turned out that this form is absolutely stable under these conditions.

The A-form has the best stability of all forms studied, and it can be reproducibly be produced, even on larger scales:

### Preparation of Q702 HCl salt on small scale

10.0 g of Q702 freebase was dissolved in acetone and HCl solution was added in the specified amounts as indicated in the below table 25 to prepare a slurry of Q702 in acetone and HCl/water. After filtration and drying, the crystal pattern/form was turned to be form A.

**Table 25: Preparation of Q702 HCl salt on small scale**

| **Lot/ Batch #** | **Starting Materials** | | | **Wet cake** | **Cake of Q702 HCl salt** | | |
|---|---|---|---|---|---|---|---|
| | **Q702 freebase** | **Solvent** | **35% HCl** | **XRPD** | **Purity** | **assay** | **XRPD (Dry cake)** |
| PS03764 -13-N | 10.0 g | Acetone (4 V); | 5.9 g | Form A | N:99.9% | 98.74% | Form A |
| | | H₂O (16 V) | | | | | |

### Preparation of Q702 HCl salt on intermediate scale

72 g of Q702 freebase was dissolved in acetone and HCl solution was added in the specified amounts as indicated in the table 26 to prepare a slurry of Q702 in acetone and HCl/water. XRPD of wet cake indicated form C was obtained. After filtration and drying, 69.3 g of Q702 HCl salt was obtained as form C with 99.8% HPLC purity in 88.3% assay yield.

**Table 26: Preparation of Q702 HCl salt on intermediate scale**

| **Lot/ Batch #** | **Starting Materials** | | | | **wet cake** | **Cake of Q702 HCl salt** | | | | |
|---|---|---|---|---|---|---|---|---|---|---|
| | **Q702 freebase** | **Solvent** | **Aq. HCl (4M)** | **H₂O** | **XRPD** | **Wt** | **Purity** | **assay** | **KF** | **XRPD (Dry cake)** |
| PS03606 -47-N | Net: 71.9 g | Acetone (4 V) | 43.2 g 3 eq. | 13 V | Form C | 69.3 g | N:99.8% | 97.9% | 3.2% | Form C |
| | | H₂O (1 V) | In2V H₂O | | | | | | | |

Hence, there appears to be scale-related or other factors that favour one form over the other. The inventors therefore surmised that there might be a change of crystal form during the crystallization process or some interconversion between the two forms that might depend on various factors such as addition rate(s) of HCl solution etc. To test this further, the inventors conducted various crystallization experiments with varying conditions, as will be outlined in the following.

### Experiment A: Tracking the change of crystal form during crystallization process

Five lots of 5 g each of Q702 freebase were used to prepare the corresponding HCl salt in crystallization experiments, in which crystallization experiments the formation and/or change of the respective crystal form(s) was tracked. For this, the addition time of aq. HCl was prolonged to 6 h. During the addition of aq. HCl, the product Q702 HCl salt would be precipitated with a certain Form, which was checked by XRPD. During crystallization, in various experiments, form A was seeded into the reaction, to see whether such interim addition/seeding of form A would have any influence on the final form achieved. The various experiments, the reaction conditions and the results are summarized in the following table 27.

**Table 27: Crystallization experiments with and without crystal seeding**

| **Lot/ Batch#/Experiment No.** | **Experimental process** | **XRPD of final product** |
|---|---|---|
| PS03606-50-N-A/Experiment 1 | 1) Dissolve Q702 freebase (5 g) in acetone (4 V, 20 mL); | Form A See also figure 18 |
| | 2) Add water (1 V, 5 mL) at 25-30 °C | |
| | 3) Add the diluted HCl solution (half) [35% HCl (3 eq., 0.58 X, 2.9 g) in water (2 V, 10 mL)] drop wise slowly at 25-30 °C over 3 h. | |
| | 4) Pull a sample for XRPD. (PS03606-50-N-A-1) | |
| | 5) Add another half of the diluted HCl solution slowly at 25-30 °C over 3 h. | |
| | 6) Pull a sample for XRPD. (PS03606-50-N-A-2) | |
| | 7) Stir reaction at 25-30 °C for 30-60 min. | |
| | 8) Add water (13 V) slowly at 25-30 °C over 16 h. | |
| | 9) Pull a sample for XRPD. (PS03606-50-N-A-3) | |
| | 10) Stir reaction at 25-30 °C for 20 h. | |
| | 11) Pull a sample for XRPD (PS03606-50-N-A-4) | |
| PS03606-50-N-B/Experiment 2 | 1) Dissolve Q702 freebase (5 g) in acetone (4 V, 20 mL); | Form A See also figure 19 |
| | 2) Add water (1 V, 5 mL) at 25-30 °C | |
| | 3) Add the diluted HCl solution (half) [35% HCl (3 eq., 0.58 X, 2.9 g) in water (3 V, 15 mL)] drop wise slowly at 25-30 °C over 3 h. | |
| | 4) Pull a sample for XRPD. (PS03606-50-N-B-1) | |
| | 5) Add another half of the diluted HCl solution slowly at 25-30 °C over 3 h. | |
| | 6) Pull a sample for XRPD. (PS03606-50-N-B-2) | |
| | 7) Stir reaction at 25-30 °C for 30-60 min. | |
| | 8) Add water (12 V) slowly at 25-30 °C over 16 h. | |
| | 9) Pull a sample for XRPD. (PS03606-50-N-B-3) | |
| | 10) Stir reaction at 25-30 °C for 20 h. | |
| | 11) Pull a sample for XRPD (PS03606-50-N-B-4) | |
| PS03606-50-N-C/Experiment 3 | 1) Dissolve Q702 freebase (5 g) in acetone (4 V, 20 mL); | Form A See also figure 20 |
| | 2) Add water (1 V, 5 mL) at 25-30 °C | |
| | 3) Add the diluted HCl solution (half) [35% HCl (3 eq., 0.58 X, 2.9 g) in water (2 V, 10 mL)] drop wise slowly at 25-30 °C over 3 h. | |
| | 4) Add Q702 HCl salt Form A as seed (0.05 g, Form A) into reaction. | |
| | 5) Stir reaction at 25-30 °C for 30-60 min. | |
| | 6) Pull a sample for XRPD. (PS03606-50-N-C-1) | |
| | 7) Add another half of the diluted HCl solution slowly at 25-30 °C over 3 h. | |
| | 8) Pull a sample for XRPD. (PS03606-50-N-C-2) | |
| | 9) Stir reaction at 25-30 °C for 30-60 min. | |
| | 10) Add water (13 V) slowly at 25-30 °C over 16 h. | |
| | 11) Pull a sample for XRPD. (PS03606-50-N-C-3) | |
| | 12) Stir reaction at 25-30 °C for 20 h. | |
| | 13) Pull a sample for XRPD (PS03606-50-N-C-4) | |
| PS03606-50-N-D/Experiment 4 | 1) Dissolve Q702 freebase (5 g) in acetone (4 V, 20 mL); | Form A See also figure 21 |
| | 2) Add water (1 V, 5 mL) at 25-30 °C | |
| | 3) Add the diluted HCl solution (half) [35% HCl (3 eq., 0.58 X, 2.9 g) in water (2 V, 10 mL)] drop wise slowly at 25-30 °C over 3 h. | |
| | 4) Pull a sample for XRPD. (PS03606-50-N-D-1) | |
| | 5) Add another half of the diluted HCl solution slowly at 25-30 °C over 3 h. | |
| | 6) Pull a sample for XRPD. (PS03606-50-N-D-2) | |
| | 7) Add Q702 HCl salt Form A as seed (0.05 g, Form A) into reaction. | |
| | 8) Stir reaction at 25-30 °C for 30-60 min. | |
| | 9) Pull a sample for XRPD. (PS03606-50-N-D-3) | |
| | 10) Add water (13 V) slowly at 25-30 °C over 16 h. | |
| | 11) Pull a sample for XRPD. (PS03606-50-N-D-4) | |
| | 12) Stir reaction at 25-30 °C for 30-60 min. | |
| | 13) Pull a sample for XRPD (PS03606-50-N-D-5) | |
| PS03606-50-N-E/Experiment 5 | 1) Dissolve Q702 freebase (5 g) in acetone (4 V, 20 mL); | Form C See also figure 22 |
| | 2) Add water (1V, 5 mL) at 25-30 °C | |
| | 3) Add the diluted HCl solution (half) [35% HCl (3 eq., 0.58 X, 2.9 g) in water (2 V, 10 mL)] drop wise slowly at 25-30 °C over 3 h. | |
| | 4) Pull a sample for XRPD. (PS03606-50-N-E-1) | |
| | 5) Add another half of the diluted HCl solution slowly at 25-30 °C over 3 h. | |
| | 6) Pull a sample for XRPD. (PS03606-50-N-E-2) | |
| | 7) Stir reaction at 25-30 °C for 30-60 min. | |
| | 8) Add water (13 V) slowly at 25-30 °C over 16 h. (Add Q702 HCl salt Form A as seed (0.05 g, Form A into reaction after adding the half of the water) | |
| | 9) Pull a sample for XRPD. (PS03606-50-N-E-3) | |
| | 10) Stir reaction at 25-30 °C for 20 h. | |
| | 11) Pull a sample for XRPD (PS03606-50-N-E-4) | |

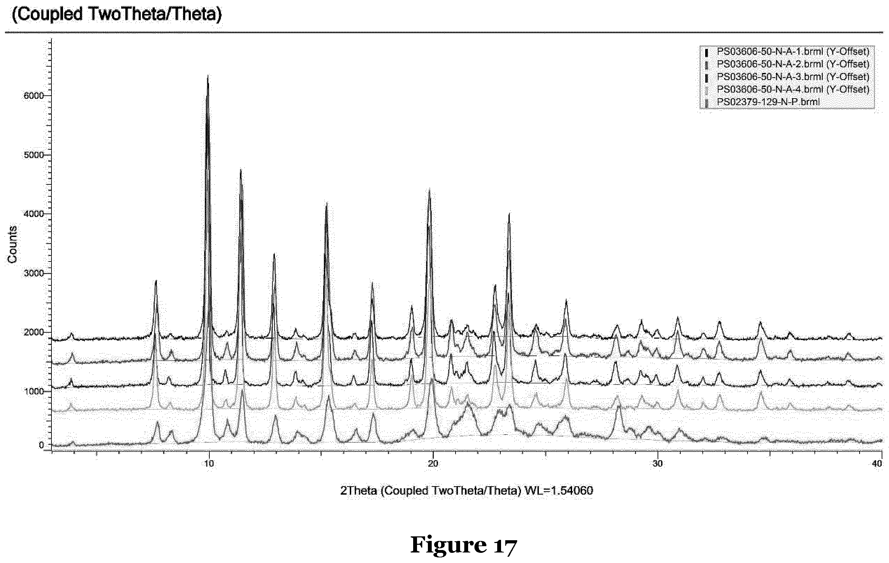

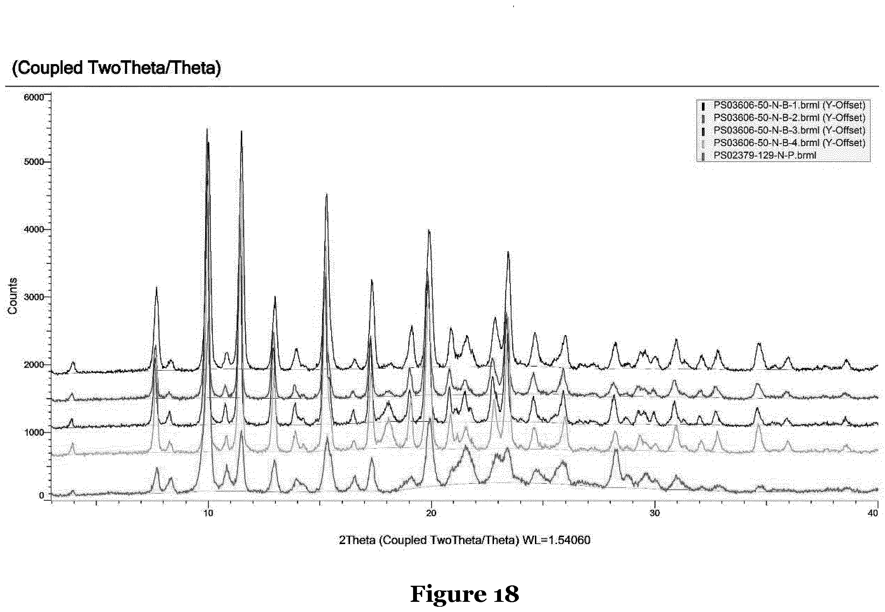

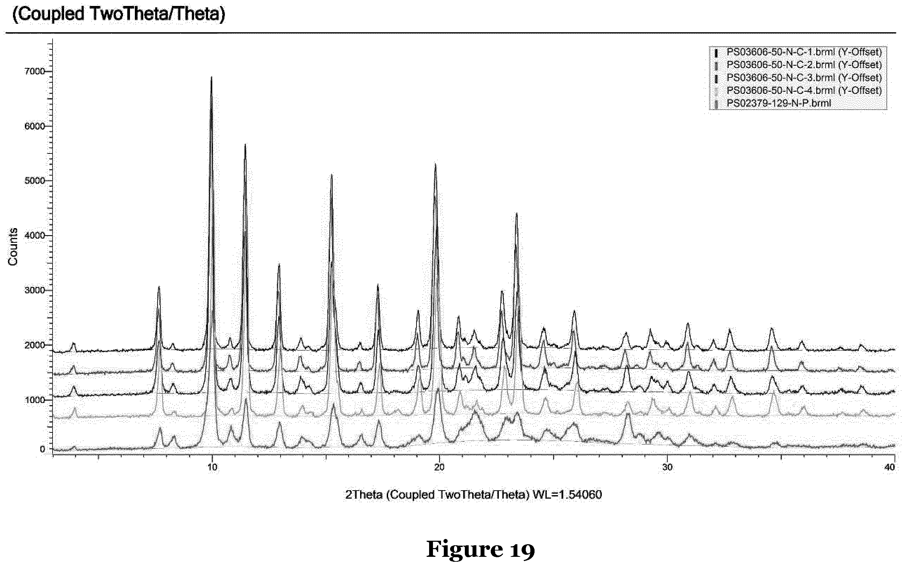

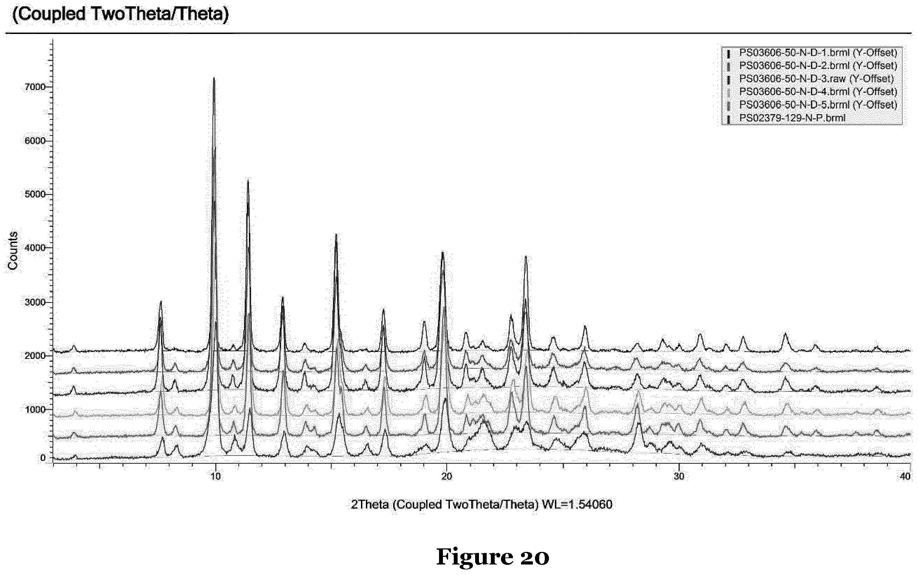

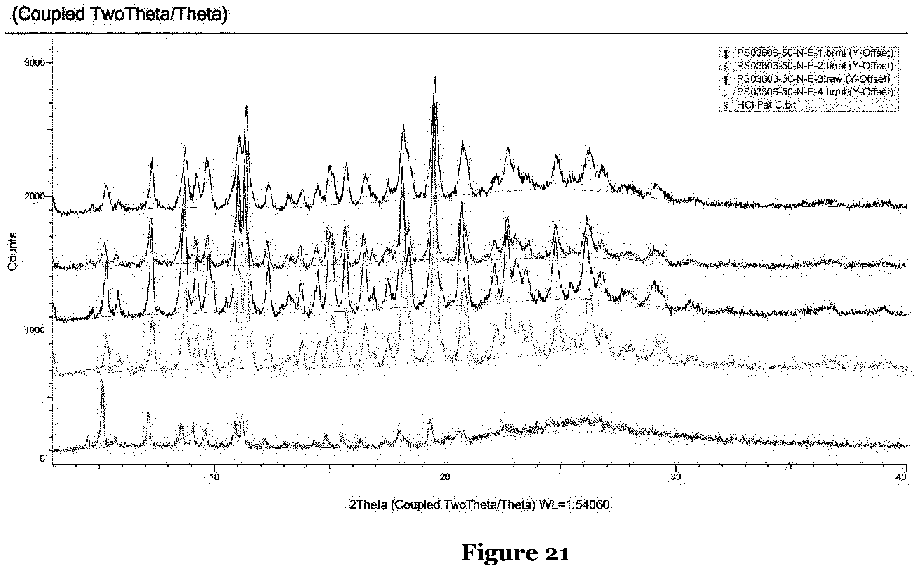

The XRPD spectra at the various stages of Experiments 1 - 5 are shown in Figures 17-21. The traces shown in Figures 17 - 20 show the various stages of the respective experiments 1 - 4 of the table 27, with an additional reference trace ("labelled as "PS02379-129-N-P.brml") of a reference XRPD spectrum of form A as the lowest trace on each figure 17- 20. In figure 21, the corresponding reference trace ("labelled as "HCl Pat C.txt") of a reference XRPD spectrum of form C is also shown as the lowest trace in this figure 21. It turns out that on this scale, under the indicated reaction conditions, Form A seems to be favoured in Experiments 1 - 4. In Experiment 5, form C is favored and seems to have been present early on in the reaction.

### Experiment B: Studying the inter-conversion of form A and form C

To test whether there is some interconversion between the different crystal forms, the inter-conversion of crystal forms was studied by stirring 50 mg of form A and 50 mg of form C into mother liquor (lot 1 in form A's mother liquor; lot 2 in form C's mother liquor). Both gave target form A and it can be concluded that under these conditions (PS03606-51-N-1-P/ Experiment 6, PS03606-51-N-2-P/ Experiment 7), form A is more stable.

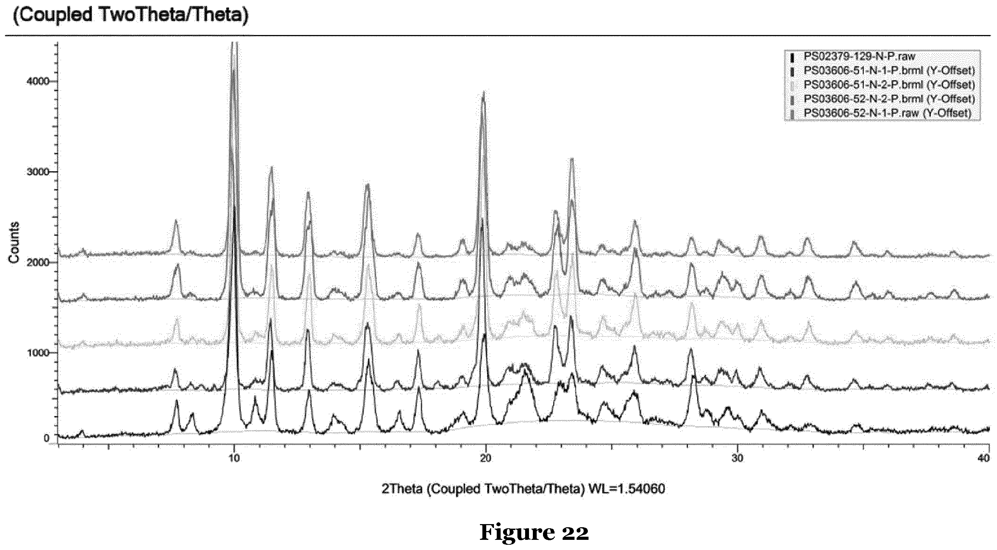

Furthermore, two additional experiments were performed to evaluate if form C can be converted to form A: In the first experiment, form C was subjected to recrystallization by dissolving form C in acetone/H₂O=1:1 (14 V), and adding water (21 V) to precipitate a solid at 20-25 °C (PS03606-52-N-1-P/ Experiment 8). In a second experiment, form C was prepared as a hot slurry in acetone/H₂O=2:8 (10 V) at 40 °C (PS03606-52-N-2-P/ Experiment 9). XRPD of both experiments indicated that target form A was the result of both experiments. (Figure 22)

In figure 22, the corresponding reference trace ("labelled as "PS02379-129-N-P.brml") of a reference XRPD spectrum of form A is also shown as the lowest trace in this figure 22.

Form A of Q702 HCl salt was tested for long term stability to 24 months. XRPD results indicated that Form A is stable to 24 months (Table 28).

**Table 28: Stability results of Form A of Q702 HCl salt**

| **Condition** | **25±2 °C/60±5%RH/****Figure 25** | | | **40 ± 2 °C/75** ± **5%RH/Figure 26** |
|---|---|---|---|---|
| **Testing Items** | **Initial** | **12M** | **24M** | **6M** |
| **Purity** | 99.9% | 100.0% | 99.9% | 99.9% |
| **Assay** | 95.9% | 94.0% | 94.6% | 94.8% |
| **XRPD** | Consistent with reference standard | Consistent with reference standard | Consistent with reference standard | Consistent with reference standard |

### Example 4

### Pharmacokinetic studies

In this example, pharmacokinetic studies are performed using the form A of the HCl-salt of Q702 and the free base (FB) thereof in plasma, following oral administration of such form A and free base to male SD rats.

Appropriate amounts of the form A of Q702 HCl or of free base were accurately weighed and mixed with appropriate volume of vehicle (water) to get a clear solution or uniform suspension. Male SD rats were dosed with a nominal dose of 30 mg/kg body weight. After dosing, 0.2 mL blood was collected per time point at time points 0 h, 0.083 h, 0.25 h, 0.5 h, 1 h, 2 h, 4 h, 8 h, 12 h, and 24 h. For such blood collection, blood was collected from the jugular vein or other suitable site of each animal into pre-chilled plastic microcentrifuged tubes containing 5 µL of 1000 IU heparin Na as anti-coagulant. Blood samples were processed for plasma by centrifugation at approximately 4 °C, 3200 g for ten minutes. Plasma was collected and transferred into pre-labelled polypropylene tubes, snap-frozen over dry ice and kept at -60 °C or lower until LC-MS/MS-analysis was performed. LC-MS/MS methodology was performed using a calibration curve with at least 6 non-zero calibration standards.

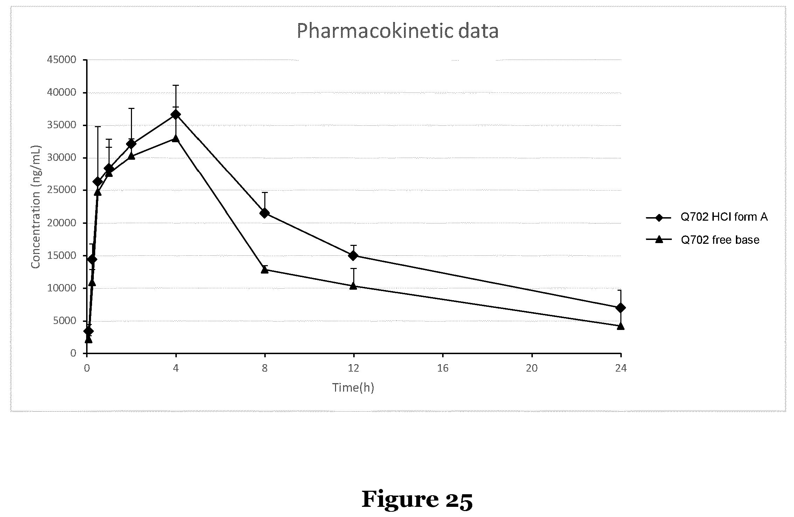

Plasma concentrations versus time were plotted and analysed by non-compartmental approaches using the Phoenix WinNonlin 6.3 software program. Related pharmacokinetic parameters were calculated according to dosing route, e. g. Cₘₐₓ, Tₘₐₓ, T_{1/2}, and AUC₍₀₋ₜ₎, for oral administration (shown in table 29 and figure 25).

**Table 29: Pharmacokinetic data**

| | **Cₘₐₓ (ng/mL)** | **Tₘₐₓ (h)** | **T_{1/2} (h)** | **AUC₀₋ₗₐₛₜ (ng.h/mL)** |
|---|---|---|---|---|
| **Q702 HCl form A (ng/mL)** | 37017 | 2,83 | 10,3 | 429743 |
| **Q702 free base (ng/mL)** | 33766 | 3,33 | 10,9 | 323390 |

Looking at the mean AUC-values, it appears that the form A of the HCl-salt of Q702 has a plasma exposure level which is 33% higher than the plasma exposure level of the free base.

Using the relative bioavailability which is calculated as:$Relative bioavailability \left(%\right) = \left(AUC_test/AUC_ref\right) \times \left(dose_ref/dose_test\right) \times 100$ wherein test = form A of HCl-salt and ref = free base of Q702

It emerges that the relative bioavailability of form A of HCl-salt of Q702 is 144.2% of the bioavailability of the free base. This huge difference in bioavailability is entirely surprising.

## Claims

1. Compound N-(5-((6,7-dimethoxyquinolin-4-yl)oxy)pyridin-2-yl)-1-propyl-4-(2,2,2-trifluoroethoxy)-1H-pyrazole-3-carboxamide hydrochloride having the formula 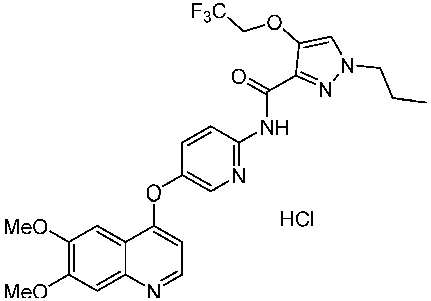 and having at least six peaks from a first set of peaks in an X-ray Powder Diffraction (XRPD) spectrum obtained by irradiation with Cu-Kα - radiation (Cu-Kα), said first set of peaks being:
8.2 ° 2θ, 9.9° 2θ, 11.4 ° 2θ, 15.2° 2θ, 15.4 ° 2θ, 19.9 ° 2θ, 21.4 ° 2θ, 21.7 ° 2θ, 22.9 ° 2θ, 23.3 ° 2θ, 25.5° 2θ, 25.7 ° 2θ, 25·9 ° 2θ, 28.2 ° 2θ, and 28.7 ° 2θ, ± 0.2 ° 2θ.

2. The compound according to claim 1, having at least two peaks from a second set of peaks in an X-ray Powder Diffraction (XRPD) spectrum obtained by irradiation with Cu-Kα - radiation (Cu-Kα), said second set of peaks being:
3.8 ° 2θ, 7.6 ° 2θ, 10.7 ° 2θ, 12.9 ° 2θ, 16.5 ° 2θ, 17.2 ° 2θ, 24.6 ° 2θ, 24.8 ° 2θ, 29.5 ° 2θ, 29.9 ° 2θ, and 31.1 ° 2θ, ± 0.2 ° 2θ;
wherein, preferably, said compound has at least three peaks or at least four peaks from said second set of peaks ("form A" of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride).

3. The compound according to any of claims 1 - 2, wherein said compound has all of the peaks from said first set of peaks and/or from said second set of peaks ("form A" of N-(5-((6,7-dimethoxyquinolin-4-yl)oxy)pyridin-2-yl)-1-propyl-4-(2,2,2-trifluoroethoxy)-1H-pyrazole-3-carboxamide hydrochloride).

4. The compound according to any of the foregoing claims having an XRPD spectrum obtained by irradiation with Cu-Kα - radiation (Cu-Kα) and as shown hereafter ("form A" of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride):

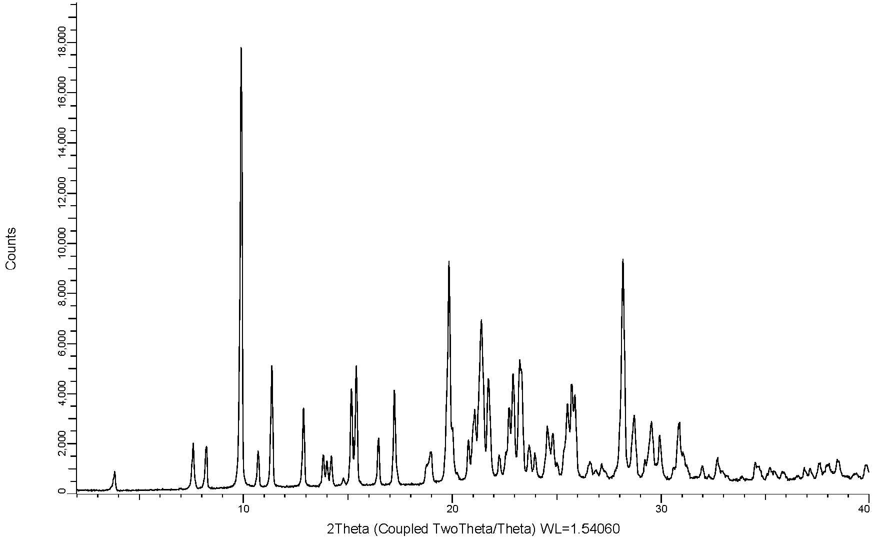

5. The compound according to any of the foregoing claims, having a differential scanning calorimetry (DSC) thermogram showing an endotherm having an onset temperature in the range of from 115 °C to 150 °C, preferably in the range of from 135 °C to 148 °C, more preferably in the range of from 135 °C to 146 °C, even more preferably in the range of from 140 °C to 146 °C, even more preferably in the range of from 144 °C to 146 °C, even more preferably at approximately 145 °C, most preferably at approximately 145.8 °C.

6. The compound according to claim 5, having a differential scanning calorimetry (DSC) thermogram as shown hereafter:

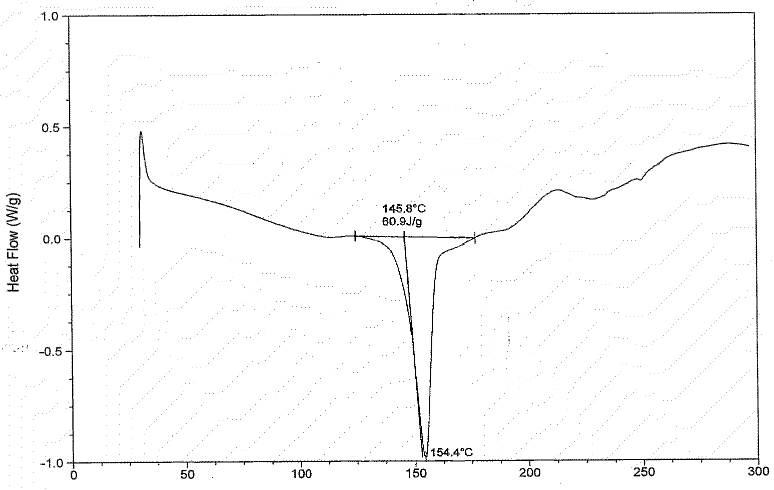

7. The compound according to any of the foregoing claims, being produced by a method comprising the steps:
- Providing, in any order, a defined amount of N-(5-((6,7-dimethoxyquinolin-4-yl)oxy)pyridin-2-yl)-1- propyl-4-(2,2,2-trifluoroethoxy)-1H-pyrazole-3-carboxamide free base and a defined amount of hydrochloric acid, such that said free base and said hydrochloric acid are provided in a stoichiometric ratio of 1:1;
- dissolving the freebase in a suitable solvent or solvent mixture, selected from methanol, ethanol, tetrahydrofuran (THF), acetone, water and a mixture of water with any of methanol, ethanol, tetrahydrofuran (THF) and acetone; and, optionally, adding 1 - 5 reaction volumes of water;
- adding approximately half of the defined amount of said hydrochloric acid;
- Adding a seeding amount of form A of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride, as defined in any of claims 1 - 4; thereby promoting formation and precipitation of form A;
- Adding the other half of the defined amount of said hydrochloric acid over a period of from 30 min to 5 h;
- Stirring for a defined period of from 30 min to 2h, preferably from 30 min to 1h;
- Adding water in an amount of 5 to 15 reaction volumes, preferably approximately 10 reaction volumes, over a period of 1 - 20 h, preferably over a period of 2 - 10 h;.
optionally isolating precipitated form A, preferably by filtration, sieving or solvent evaporation.

8. A method for making the compound as defined in any of claims 1 - 7, said method comprising the steps:
- Providing, in any order, a defined amount of N-(5-((6,7-dimethoxyquinolin-4-yl)oxy)pyridin-2-yl)-1- propyl-4-(2,2,2-trifluoroethoxy)-1H-pyrazole-3-carboxamide free base and a defined amount of hydrochloric acid, such that said free base and said hydrochloric acid are provided in a stoichiometric ratio of 1:1;
- dissolving the freebase in a suitable solvent or solvent mixture, selected from methanol, ethanol, tetrahydrofuran (THF), acetone, water and a mixture of water with any of methanol, ethanol, tetrahydrofuran (THF) and acetone; and, optionally, adding 1 - 5 reaction volumes of water;
- adding approximately half of the defined amount of said hydrochloric acid;
- Adding a seeding amount of form A of N-(5-((6,7-dimethoxyquinolin-4-yl)oxy)pyridin-2-yl)-1- propyl-4-(2,2,2-trifluoroethoxy)-1H-pyrazole-3-carboxamide hydrochloride, as defined in any of claims 1 - 4; thereby promoting formation and precipitation of form A;
- Adding the other half of the defined amount of said hydrochloric acid over a period of from 30 min to 5 h;
- Stirring for a defined period of from 30 min to 2 h, preferably from 30 min to 1h;
- Adding water in an amount of 5 to 15 reaction volumes, preferably approximately 10 reaction volumes, over a period of 1 - 20 h, preferably over a period of 2 - 10 h;.
- optionally isolating precipitated form A, preferably by filtration, sieving or solvent evaporation.

9. A pharmaceutical composition comprising at least one compound according to any of claims 1-7, together with at least one pharmaceutically acceptable carrier, excipient and/or diluent.

10. The pharmaceutical composition according to claim 9, further comprising at least one other pharmaceutically active agent.

11. The compound according to any of claims 1-7 or the pharmaceutical composition according to any of claims 9 - 10, for use in the treatment of a disorder selected from hyperproliferative disorders, inflammatory disorders and neurodegenerative disorders.

12. The compound or pharmaceutical composition for use according to claim 11, wherein said hyperproliferative disorder is a cancer, preferably a cancer selected from adenocarcinoma, acoustic neuroma, acute lymphoblastic leukemia, acute myeloid leukemia, adrenocortical carcinoma, aids-related cancers, aids-related lymphoma, anal cancer, appendix cancer, astrocytomas, atypical teratoid/rhabdoid tumor, ampullary carcinoma, basal cell carcinoma, bile duct cancer, bladder cancer, bone cancer, osteosarcoma and malignant fibrous histiocytoma, brain stem glioma, brain tumor, central nervous system atypical teratoid/rhabdoid tumor, craniopharyngioma, ependymoblastoma, ependymoma, medulloblastoma, medulloepithelioma, pineal parenchymal tumors of intermediate differentiation, supratentorial primitive neuroectodermal tumors and pineoblastoma, brain and spinal cord tumors, breast cancer, urachal tumors, burkitt lymphoma, carcinoid tumor, choroidal melanoma, gastrointestinal cancer, central nervous system lymphoma, cervical cancer, corpus cancer, chordoma, chronic lymphocytic leukemia, chronic myelogenous leukemia, chronic myeloproliferative disorders, colon cancer, colorectal cancer, cutaneous t-cell lymphoma, desmoid tumor, mycosis fungoides, endometrial cancer, esophageal cancer, esthesioneuroblastoma, Ewing sarcoma family of tumors, extracranial germ cell tumor, extragonadal germ cell tumor, extrahepatic bile duct cancer, ear tumors, intraocular melanoma, retinoblastoma, gallbladder cancer, gastric cancer, gastrointestinal carcinoid tumor, gastrointestinal stromal tumor, gastrointestinal stromal cell tumor, gynecologic tumors, ovarian germ cell tumor, gestational trophoblastic tumor, glioma, gallbladder carcinomas, hairy cell leukemia, head and neck cancer, heart cancer, hepatocellular cancer, histiocytosis, hypopharyngeal cancer, hematologic neoplasias, islet cell tumors (endocrine pancreas), renal cell cancer, kidney cancer, langerhans cell histiocytosis, laryngeal cancer, leukemia, lip and oral cavity cancer, liver cancer, lung cancer, non-small cell lung cancer, small intestinal tumors, small cell lung cancer, hodgkin lymphoma, non-hodgkin lymphoma, primary central nervous system lymphoma, macroglobulinemia, malignant fibrous histiocytoma of bone and osteosarcoma, melanoma, merkel cell carcinoma, mesothelioma, metastatic squamous neck cancer with occult primary, spinalioms, multiple endocrine neoplasia syndromes, myelodysplastic syndromes, myelodysplastic/myeloproliferative neoplasms, myeloid leukemia, multiple myeloma, myeloproliferative disorders, nasal cavity and paranasal sinus cancer, nasopharyngeal cancer, neuroblastoma, oral cavity cancer, oropharyngeal cancer, osteosarcoma and malignant fibrous histiocytoma of bone, ovarian cancer, ovarian epithelial cancer, ovarian low malignant potential tumor, oligodendroglioma, plasmacytomas, pancreatic cancer, papillomatosis, parathyroid cancer, penile cancer, pharyngeal cancer, pituitary tumor, plasma cell neoplasm/multiple myeloma, pleuropulmonary blastoma, prostate cancer, rectal cancer, renal cell cancer, transitional cell cancer, respiratory tract cancer, rhabdomyosarcoma, salivary gland cancer, sarcoma, skin testis cancer, ewing sarcoma, kaposi sarcoma, uterine sarcoma, non-melanoma skin cancer, melanoma skin cancer, skin carcinoma, small intestine cancer, soft tissue sarcoma, squamous cell carcinoma, squamous neck cancer, stomach cancer, soft tissue tumors, testicular cancer, throat cancer, thymoma and thymic carcinoma, thyroid cancer, transitional cell cancer of the renal pelvis and ureter, trophoblastic tumor, testicle cancer, gestational cancer, urologic tumors, ureter and renal pelvis cancer, urethral cancer, urothelial carcinoma, uterine cancer, vaginal cancer, vulvar cancer, waldenström macroglobulinemia and wilms tumor, tumors that cause effusions in potential spaces of the body, pleural effusions, pericardial effusions, peritoneal effusion aka ascites, giant cell tumor (GCT), GCT of bone, pigmented villonodular synovitis (PVNS), tenosynovial giant cell tunor (TGCT), TGCT of tendon sheath (TGCT-TS).

13. The compound or pharmaceutical composition for use according to claim 11, wherein said inflammatory disorder is selected from osteoarthritis, inflammatory bowel syndrome, tramsplant rejection, systemic lupus erythematosis, ulcerative colitis, crohn's disease, chronic obstructive pulmonary disease, emphysema, Kawasaki's Disease, hemophagocytic syndrome (macrophage activation syndrome), multicentric reticulohistiocytosis, atherosclerosis, primary progressive multiple sclerosis, tenpsy Type I diabetes, Type I1 diabetes, insulin resistance, hyperglycemia, obesity, lipolysis, hypcreosinophilia, osteoporosis, increased risk of fracture, Paget's disease, hypercalcemia, infectionmediated osteolysis (e.g. osteomyelitis), peri-prosthetic or wear-debris-mediated osteolysis, endometriosis, inflammatory pain, chronic pain, and bone pain.

14. The compound or pharmaceutical composition for use according to claim 11, wherein said neurodegenerative disorder is selected from Binswanger type dementia, prosencephaly, microcephaly, cerebral palsy, congenital hydrocephalus, abdominal dropsy, progress supranuclear palsy, glaucoma, Wilson disease, Alzheimer's disease and other dementias, Parkinson's disease (PD) and PD-related disorders, multi infarct dementia, Frontotemporal dementia, pseudo-dementia, Prion disease, Motor neuron diseases, Huntington's disease, spinocerebellar ataxia, and spinal muscular atrophy.

15. The compound or pharmaceutical composition for use according to any of claims 11 - 14, wherein said use is in combination with another pharmaceutically active drug or therapy, in particular radiation therapy, chemotherapy agents, targeted drugs and immune check point inhibitor drugs.

## Patentansprüche

1. Verbindung N-(5-((6,7-Dimethoxychinolin-4-yl)oxy)pyridin-2-yl)-1-propyl-4-(2,2,2-trifluoroethoxy)-1H-pyrazol-3-carboxamid-hydrochlorid
mit der Formel 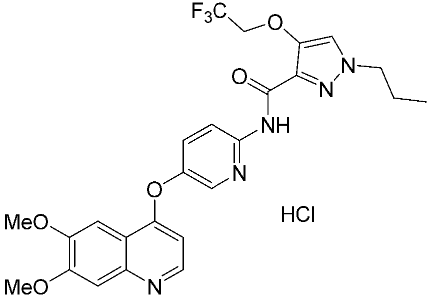 und mit wenigstens sechs Signalen aus einem ersten Satz von Signalen in einem Röntgenpulverdiffraktions (XRPD)-Spektrum, erhalten durch Bestrahlung mit Cu-Kα-Strahlung (Cu-Kα), wobei der erste Satz an Signalen ist:
8.2 ° 2θ, 9.9° 2θ, 11.4 ° 2θ, 15.2° 2θ, 15.4 ° 2θ, 19.9 ° 2θ, 21.4 ° 2θ, 21.7 ° 2θ, 22.9 ° 2θ, 23.3 ° 2θ, 25.5 ° 2θ, 25.7 ° 2θ, 25.9 ° 2θ, 28.2 ° 2θ, and 28.7 ° 2θ, ± 0.2 ° 2θ.

2. Verbindung nach Anspruch 1 mit wenigstens zwei Signalen aus einem zweiten Satz von Signalen in einem Röntgenpulverdiffraktions (XRPD)-Spektrum, erhalten durch Bestrahlung mit Cu-Kα-Strahlung (Cu-Kα), wobei der zweite Satz an Signalen ist:
3.8 ° 2θ, 7.6 ° 2θ, 10.7 ° 2θ, 12.9 ° 2θ, 16.5 ° 2θ, 17.2 ° 2θ, 24.6 ° 2θ, 24.8 ° 2θ, 29.5 ° 2θ, 29.9 ° 2θ, and 31.1 ° 2θ, ± 0.2 ° 2θ;
wobei bevorzugt die Verbindung wenigstens drei Signale oder wenigstens vier Signale aus dem zweiten Satz von Signalen hat ("Form A" von N-(5-((6,7-Dimethoxychinolin-4-yl)oxy)pyridin-2-yl)-1-propyl-4-(2,2,2-trifluoroethoxy)-1H-pyrazol-3-carboxamid-hydrochlorid).

3. Verbindung nach einem der Ansprüche 1-2, wobei die Verbindung alle Signale aus dem ersten Satz von Signalen und/oder aus dem zweiten Satz von Signalen hat ("Form A" von N-(5-((6,7-Dimethoxychinolin-4-yl)oxy)pyridin-2-yl)-1-propyl-4-(2,2,2-trifluoroethoxy)-1H-pyrazol-3-carboxamid-hydrochlorid).

4. Verbindung nach einem der vorangehenden Ansprüche mit einem XRPD-Spektrum, erhalten durch Bestrahlung mit Cu-Kα-Strahlung (Cu-Kα) und wie hiernach gezeigt ("Form A" von N-(5-((6,7-Dimethoxychinolin-4-yl)oxy)pyridin-2-yl)-1-propyl-4-(2,2,2-trifluoroethoxy)-1H-pyrazol-3-carboxamid-hydrochlorid):

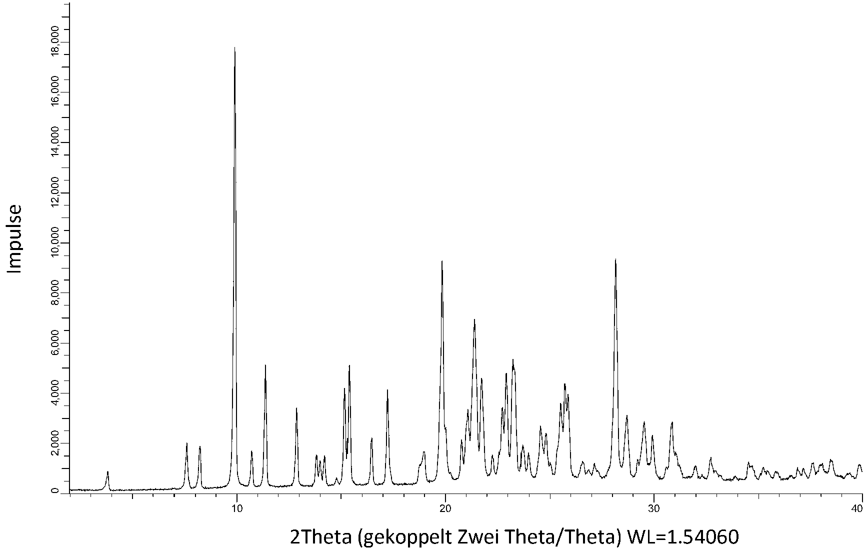

5. Verbindung nach einem der vorangegangenen Ansprüche, mit einem Differential-Scanning-Kalorimetrie (DSC)-Thermogramm, das eine Endotherme mit einer Einsetztemperatur im Bereich von 115°C bis 150°C zeigt, bevorzug im Bereich von 135°C bis 148°C, bevorzugter im Bereich von 135°C bis 146°C, noch bevorzugter im Bereich von 140°C bis 146°C, noch bevorzugter im Bereich von 144°C bis 146°C, noch bevorzugter bei näherungsweise von 145°C, am bevorzugtesten bei näherungsweise bei 145,8°C.

6. Verbindung nach Anspruch 5 mit einem Differential-Scanning-Kalorimetrie (DSC)-Thermogramm, wie hiernach gezeigt:

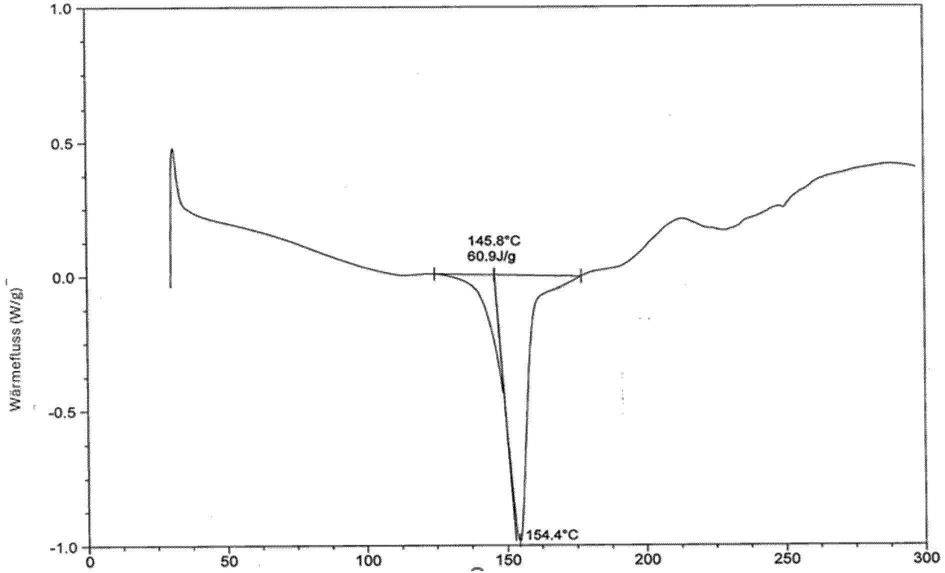

7. Verbindung nach einem der vorangehenden Ansprüche, hergestellt durch ein Verfahren, umfassend die Schritte:
- Bereitstellen, in einer beliebigen Reihenfolge, einer definierten Menge der freien Base von N-(5-((6,7-Dimethoxychinolin-4-yl)oxy)pyridin-2-yl)-1-propyl-4-(2,2,2-trifluoroethoxy)-1H-pyrazol-3-carboxamid und einer definierten Menge an Salzsäure, so dass die freie Base und die Salzsäure in einem stöchiometrischen Verhältnis von 1:1 bereitgestellt werden;
- Auflösen der freien Base in einem geeigneten Lösungsmittel oder einer Lösungsmittelmischung, ausgewählt aus Methanol, Ethanol, Tetrahydrofuran (THF), Aceton, Wasser und einer Mischung von Wasser mit einem von Methanol, Ethanol, Tetrahydrofuran (THF) und Aceton; und wahlweise Zugeben von 1-5 Reaktionsvolumina Wasser;
- Zugeben von näherungsweise der Hälfte der definierten Menge an Salzsäure;
- Zugeben einer Startermenge der Form A von N-(5-((6,7-Dimethoxychinolin-4-yl)oxy)pyridin-2-yl)-1-propyl-4-(2,2,2-trifluoroethoxy)-1H-pyrazol-3-carboxamid-hydrochlorid, wie in einem der Ansprüche 1-4 definiert, wodurch die Bildung und Fällung von Form A gefördert wird,
- Zugeben der anderen Hälfte der definierten Menge der Salzsäure, über eine Periode von 30 Min bis 5h;
- Rühren für eine definierte Priode von 30 Min bis 2h, bevorzugt von 30 Min bis 1h;
- Zugeben von Wasser in einer Menge von 5-15 Reaktionsvolumina, bevorzugt näherungsweise 10 Reaktionsvolumina, über eine Periode von 1-20h, bevorzugt über eine Periode von 2-10h;
- wahlweise Isolieren der ausgefällten Form A, bevorzugt durch Filtration, Sieben oder Lösungsmittelverdampfung.

8. Verfahren zum Herstellen der Verbindung wie in einem der Ansprüche 1-7 definiert, wobei das Verfahren die Schritte umfasst:
- Bereitstellen in einer beliebigen Reihenfolge, einer definierten Menge der freien Base von N-(5-((6,7-Dimethoxychinolin-4-yl)oxy)pyridin-2-yl)-1-propyl-4-(2,2,2-trifluoroethoxy)-1H-pyrazol-3-carboxamid und einer definierten Menge an Salzsäure, so dass die freie Base und die Salzsäure in einem stöchiometrischen Verhältnis von 1:1 bereitgestellt werden;
- Auflösen der freien Base in einem geeigneten Lösungsmittel oder Lösungsmittelmischung, ausgewählt aus Methanol, Ethanol, Tetrahydrofuran (THF), Aceton, Wasser und einer Mischung von Wasser mit einem von Methanol, Ethanol, Tetrahydrofuran (THF) und Aceton; und wahlweise Zugeben von 1-5 Reaktionsvolumina Wasser;
- Zugeben von näherungsweise der Hälfte der definierten Menge an Salzsäure;
- Zugeben einer Startermenge der Form A von N-(5-((6,7-Dimethoxychinolin-4-yl)oxy)pyridin-2-yl)-1-propyl-4-(2,2,2-trifluoroethoxy)-1H-pyrazol-3-carboxamid-hydrochlorid, wie in einem der Ansprüche 1-4 definiert; wodurch die Bildung und Fällung von Form A gefördert wird;
- Zugeben der anderen Hälfte der definierten Menge der Salzsäure über eine Periode von 30 Min bis 5h;
- Rühren für eine definierte Periode von 30 Min bis 2h, bevorzugt von 30 Min bis 1h;
- Zugeben von Wasser in einer Menge von 5-15 Reaktionsvolumina, bevorzugt näherungsweise 10 Reaktionsvolumina, über eine Priode von 1-20h, bevorzugt über eine Periode von 2-10h;
- wahlweise Isolieren der ausgefällten Form A, bevorzugt durch Filtration, Sieben oder Lösungsmittelverdampfung.

9. Pharmazeutische Zusammensetzung umfassend mindestens eine Verbindung nach einem der Ansprüche 1-7, zusammen mit wenigstens einem pharmazeutisch akzeptablen Träger, Hilfsstoff und/oder Verdünnungsmittel.

10. Pharmazeutische Zusammensetzung nach Anspruch 9, weiterhin umfassend mindestens einen anderen pharmazeutisch aktiven Wirkstoff.

11. Verbindung nach einem der Ansprüche 1-7 oder pharmazeutische Zusammensetzung nach einem der Ansprüche 9-10, zur Verwendung bei der Behandlung einer Krankheit, ausgewählt aus hyperproliferativen Krankheiten, entzündlichen Krankheiten und neurodegenerativen Krankheiten.

12. Verbindung oder pharmazeutische Zusammensetzung zur Verwendung nach Anspruch 11, wobei die hyperproliferative Krankheit ein Krebs ist, bevorzugt ein Krebs, ausgewählt aus Adenokarzinom, Akustikusneurinom, akute lymphoblastische Leukämie, akute myeloische Leukämie, Nebennierenrindenkarzinom, AIDSassoziierte Krebserkrankungen, AIDS-assoziiertes Lymphom, Analkrebs, Blinddarmkrebs, Astrozytome, atypischer teratoider/rhabdoider Tumor, Ampullenkarzinom, Basalzellkarzinom, Gallengangskrebs, Blasenkrebs, Knochenkrebs, Osteosarkom und malignes fibröses Histiozytom, Hirnstammgliom, Hirntumor, atypischer teratoider/rhabdoider Tumor des Zentralnervensystems, Kraniopharyngeom, Ependymoblastom, Ependymom, Medulloblastom, Medulloepitheliom, parenchymale Tumoren der Zirbeldrüse mittlerer Differenzierung, supratentoriale primitive neuroektodermale Tumoren und Pineoblastom, Hirn- und Rückenmarkstumoren, Brustkrebs, Urachustumoren, Burkitt-Lymphom, Karzinoidtumor, Aderhautmelanom, Magen-Darm-Krebs, Lymphom des Zentralnervensystems, Gebärmutterhalskrebs, Corpus-Krebs, Chordom, chronische lymphatische Leukämie, chronische myeloische Leukämie, chronische myeloproliferative Erkrankungen, Dickdarmkrebs, kolorektaler Krebs, kutanes T-Zell-Lymphom, Desmoidtumor, Mycosis fungoides, Gebärmutterschleimhautkrebs, Speiseröhrenkrebs, Ästhesioneuroblastom, Tumoren der Ewing-Sarkom-Familie, extrakranielle Keimzelltumoren, extragonadale Keimzelltumoren, extrahepatischer Gallengangskrebs, Ohrtumoren, intraokulares Melanom, Retinoblastom, Gallenblasenkrebs, Magenkrebs, Karzinoidtumor des Magen-Darm-Trakts, gastrointestinaler Stromatumor, gastrointestinaler Stromazelltumor, gynäkologische Tumoren, Keimzelltumor des Eierstocks, gestationeller Trophoblasttumor, Gliom, Gallenblasenkarzinome, Haarzell-Leukämie, Kopf-Hals-Krebs, Herzkrebs, Leberzellkarzinom, Histiozytose, Hypopharynxkarzinom, hämatologische Neoplasien, Inselzelltumoren (endokrine Bauchspeicheldrüse), Nierenzellkarzinom, Nierenkrebs, Langerhans-Zell-Histiozytose, Kehlkopfkrebs, Leukämie, Lippen- und Mundhöhlenkrebs, Leberkrebs, Lungenkrebs, nicht-kleinzelliges Lungenkarzinom, Tumoren des Dünndarms, kleinzelliges Lungenkarzinom, Hodgkin-Lymphom, Non-Hodgkin-Lymphom, primäres Lymphom des Zentralnervensystems, Makroglobulinämie, malignes fibröses Histiozytom des Knochens und Osteosarkom, Melanom, Merkelzellkarzinom, Mesotheliom, metastasierendes Plattenepithelkarzinom des Halses mit okkultem Primärtumor, Spinaliome, Syndrome der multiplen endokrinen Neoplasie, myelodysplastische Syndrome, myelodysplastische/myeloproliferative Neoplasien, myeloische Leukämie, Multiples Myelom, myeloproliferative Erkrankungen, Nasenhöhlen- und Nasennebenhöhlenkrebs, Nasopharynxkarzinom, Neuroblastom, Mundhöhlenkrebs, Oropharynxkarzinom, Osteosarkom und malignes fibröses Histiozytom des Knochens, Eierstockkrebs, epithelialer Eierstockkrebs, Tumor des Eierstocks mit geringem malignem Potenzial, Oligodendrogliom, Plasmazytome, Bauchspeicheldrüsenkrebs, Papillomatose, Nebenschilddrüsenkrebs, Peniskrebs, Rachenkrebs, Hypophysentumor, Plasmazellneoplasma/Multiples Myelom, Pleuropulmonales Blastom, Prostatakrebs, Rektumkarzinom, Nierenzellkarzinom, Übergangszellkarzinom, Krebs der Atemwege, Rhabdomyosarkom, Speicheldrüsenkrebs, Sarkom, Hautkrebs, Hodenkrebs, Ewing-Sarkom, Kaposi-Sarkom, Gebärmutter-Sarkom, nicht-melanomatöser Hautkrebs, Melanom, Hautkarzinom, Dünndarmkrebs, Weichteilsarkom, Plattenepithelkarzinom, Plattenepithelkarzinom im Halsbereich, Magenkrebs, Weichteiltumoren, Hodenkrebs, Kehlkopfkrebs, Thymom und Thymuskarzinom, Schilddrüsenkrebs, Übergangszellkarzinom des Nierenbeckens und des Harnleiters, Trophoblastentumor, Hodenkrebs, Schwangerschaftskrebs, urologische Tumoren, Harnleiter- und Nierenbeckenkrebs, Harnröhrenkrebs, Urothelkarzinom, Gebärmutterkrebs, Vaginalkrebs, Vulvakrebs, Waldenström-Makroglobulinämie und Wilms-Tumor, Tumoren, die Ergüsse in potenziellen Körperräumen verursachen, Pleuraergüsse, Perikardergüsse, Peritonealerguss (auch Aszites genannt), Riesenzelltumor (GCT), GCT des Knochens, pigmentierte villonoduläre Synovitis (PVNS), tenosynovialer Riesenzelltumor (TGCT), TGCT der Sehnenscheide (TGCT-TS).

13. Verbindung oder pharmazeutische Zusammensetzung zur Verwendung nach Anspruch 11, wobei die entzündliche Erkrankung ausgewählt ist aus Arthrose, entzündliches Darmerkrankungssyndrom, Transplantatabstoßung, systemischer Lupus erythematodes, Colitis ulcerosa, Morbus Crohn, chronisch-obstruktive Lungenerkrankung, Emphysem, Kawasaki-Syndrom, hämophagozytisches Syndrom (Makrophagenaktivierungssyndrom), multizentrische Retikulohistizytose, Atherosklerose, primär progrediente Multiple Sklerose, Typ-I-Diabetes, Typ-II-Diabetes, Insulinresistenz, Hyperglykämie, Adipositas, Lipolyse, Hypereosinophilie, Osteoporose, erhöhtes Frakturrisiko, Morbus Paget, Hyperkalzämie, infektionsbedingte Osteolyse (z. B. Osteomyelitis), periprothetische oder durch Abriebpartikel verursachte Osteolyse, Endometriose, entzündliche Schmerzen, chronische Schmerzen und Knochenschmerzen.

14. Verbindung oder pharmazeutische Zusammensetzung zur Verwendung nach Anspruch 11, wobei die neurodegenerative Erkrankung ausgewählt ist aus Binswanger-Demenz, Prosenzephalie, Mikrozephalie, Zerebralparese, angeborener Hydrozephalus, Bauchwassersucht, progressive supranukleäre Lähmung, Glaukom, Morbus Wilson, Alzheimer-Krankheit und andere Demenzerkrankungen, Parkinson-Krankheit (PD) und PD-verwandte Erkrankungen, Multiinfarkt-Demenz, frontotemporale Demenz, Pseudodemenz, Prionenerkrankungen, Motoneuronerkrankungen, Huntington-Krankheit, spinozerebelläre Ataxie und spinale Muskelatrophie.

15. Verbindung oder pharmazeutische Zusammensetzung zur Verwendung nach einem der Ansprüche 11-14, wobei die Verwendung in Kombination mit einem anderen pharmazeutisch aktiven Wirkstoff oder Therapie erfolgt, insbesondere Strahlungstherapie, chemotherapeutische Mittel, zielgerichtete Arzneien und Immun-Checkpoint-Inhibitor-Arzneien.

## Revendications

1. Composé chlorhydrate de N-(5-((6,7-diméthoxyquinoléin-4-yl)oxy)pyridin-2-yl)-1-propyl-4-(2,2,2-trifluoroéthoxy)-1H-pyrazole-3-carboxamide ayant la formule 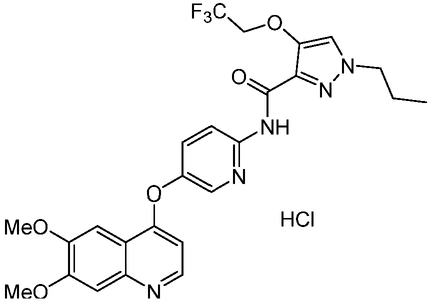 et présentant au moins six pics d'un premier ensemble de pics dans un spectre de diffraction des rayons X sur poudre (XRPD) obtenu par irradiation avec un rayonnement Cu-Kα (Cu-Kα), ledit premier ensemble de pics étant :
8,2° 2θ, 9,9° 2θ, 11,4° 2θ, 15,2° 2θ, 15,4° 2θ, 19,9° 2θ, 21,4° 2θ, 21,7° 2θ, 22,9° 2θ, 23,3° 2θ, 25,5° 2θ, 25,7° 2θ, 25,9° 2θ, 28,2° 2θ, et 28,7° 2θ, ± 0,2° 2θ.

2. Composé selon la revendication 1, présentant au moins deux pics d'un deuxième ensemble de pics dans un spectre de diffraction des rayons X sur poudre (XRPD) obtenu par irradiation avec un rayonnement Cu-Kα (Cu-Kα), ledit deuxième ensemble de pics étant :
3,8° 2θ, 7,6° 2θ, 10,7° 2θ, 12,9° 2θ, 16,5° 2θ, 17,2° 2θ, 24,6° 2θ, 24,8° 2θ, 29,5° 2θ, 29,9° 2θ, et 31,1° 2θ, ± 0,2° 2θ ;
dans lequel, de préférence, ledit composé a au moins trois pics ou au moins quatre pics dudit deuxième ensemble de pics (« forme A » de chlorhydrate de N-(5-((6,7-diméthoxyquinoléin-4-yl)oxy)pyridin-2-yl)-1-propyl-4-(2,2,2-trifluoroéthoxy)-1H-pyrazole-3-carboxamide).

3. Composé selon l'une quelconque des revendications 1 à 2, dans lequel ledit composé présente tous les pics dudit premier ensemble de pics et/ou dudit deuxième ensemble de pics (« forme A » de chlorhydrate de N-(5-((6,7-diméthoxyquinoléin-4-yl)oxy)pyridin-2-yl)-1-propyl-4-(2,2,2-trifluoroéthoxy)-1H-pyrazole-3-carboxamide).

4. Composé selon l'une quelconque des revendications précédentes ayant un spectre de XRPD obtenu par irradiation avec un rayonnement Cu-Kα (Cu-Kα) et comme indiqué ci-après (« forme A » du chlorhydrate de N-(5-((6,7-diméthoxyquinoléin-4-yl)oxy)pyridin-2-yl)-1-propyl-4-(2,2,2-trifluoroéthoxy)-1H-pyrazole-3-carboxamide) :

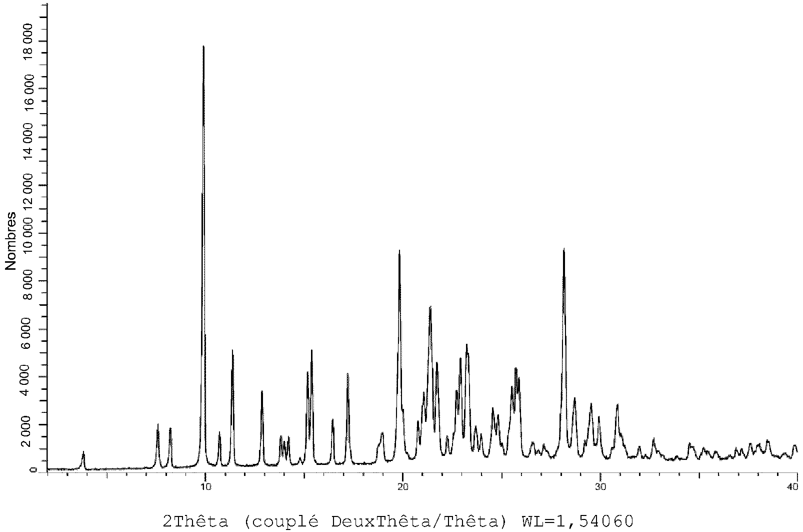

5. Composé selon l'une quelconque des revendications précédentes, ayant un thermogramme de calorimétrie différentielle à balayage (DSC) présentant un endotherme ayant une température d'apparition dans la plage allant de 115 °C à 150 °C, de préférence dans la plage allant de 135 °C à °148 C, plus préférablement dans la plage allant de 135 °C à 146 °C, encore plus préférablement dans la plage allant de 140 °C à 146 °C, encore plus préférablement dans la plage allant de 144 °C à 146 °C, encore plus préférablement à approximativement 145 °C, de manière préférée entre toutes à approximativement 145,8 °C.

6. Composé selon la revendication 5, ayant un thermogramme de calorimétrie différentielle à balayage (DSC) comme indiqué ci-après:

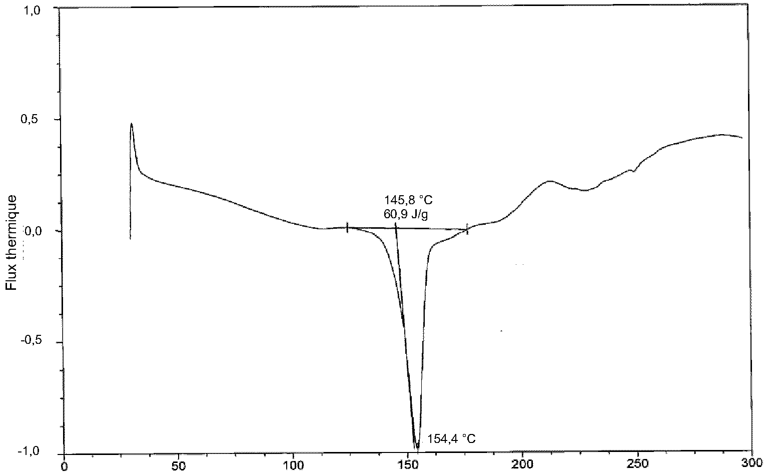

7. Composé selon l'une quelconque des revendications précédentes, qui est produit par un procédé comprenant les étapes :
- fourniture, dans un ordre quelconque, d'une quantité définie de base libre de N-(5-((6,7-diméthoxyquinoléin-4-yl)oxy)pyridin-2-yl)-1-propyl-4-(2,2,2-trifluoroéthoxy)-1H-pyrazole-3-carboxamide et d'une quantité définie d'acide chlorhydrique, de sorte que ladite base libre et ledit acide chlorhydrique soient fournis selon un rapport stœchiométrique de 1:1 ;
- dissolution de la base libre dans un solvant ou mélange de solvants approprié, choisis parmi le méthanol, l'éthanol, le tétrahydrofurane (THF), l'acétone, l'eau et un mélange d'eau avec l'un quelconque parmi le méthanol, l'éthanol, le tétrahydrofurane (THF) et l'acétone ; et, éventuellement, ajout de 1 - 5 volumes de réaction d'eau ;
- ajout d'environ la moitié de la quantité définie dudit acide chlorhydrique ;
- ajout d'une quantité d'ensemencement de la forme A du chlorhydrate de N-(5-((6,7-diméthoxyquinoléin-4-yl)oxy)pyridin-2-yl)-1-propyl-4-(2,2,2-trifluoroéthoxy)-1H-pyrazole-3-carboxamide, tel que défini selon l'une quelconque des revendications 1 à 4 ; favorisant ainsi la formation et la précipitation de la forme A ;
- ajout de l'autre moitié de la quantité définie dudit acide chlorhydrique pendant une période de 30 min à 5 h ;
- agitation pendant une durée définie de 30 min à 2 h, de préférence de 30 min à 1 h ;
- ajout d'eau en une quantité de 5 à 15 volumes de réaction, de préférence environ 10 volumes de réaction, sur une durée de 1 - 20 h, de préférence sur une durée de 2 - 10 h ;
éventuellement isolement de la forme A précipitée, de préférence par filtration, tamisage ou évaporation de solvant.

8. Procédé de préparation du composé tel que défini selon l'une quelconque des revendications 1 à 7, ledit procédé comprenant les étapes :
- fourniture, dans un ordre quelconque, d'une quantité définie de base libre de N-(5-((6,7-diméthoxyquinoléin-4-yl)oxy)pyridin-2-yl)-1-propyl-4-(2,2,2-trifluoroéthoxy)-1H-pyrazole-3-carboxamide et d'une quantité définie d'acide chlorhydrique, de sorte que ladite base libre et ledit acide chlorhydrique soient fournis selon un rapport stœchiométrique de 1:1 ;
- dissolution de la base libre dans un solvant ou mélange de solvants approprié, choisis parmi le méthanol, l'éthanol, le tétrahydrofurane (THF), l'acétone, l'eau et un mélange d'eau avec l'un quelconque parmi le méthanol, l'éthanol, le tétrahydrofurane (THF) et l'acétone ; et, éventuellement, ajout de 1 - 5 volumes de réaction d'eau ;
- ajout d'environ la moitié de la quantité définie dudit acide chlorhydrique ;
- ajout d'une quantité d'ensemencement de la forme A du chlorhydrate de N-(5-((6,7-diméthoxyquinoléin-4-yl)oxy)pyridin-2-yl)-1-propyl-4-(2,2,2-trifluoroéthoxy)-1H-pyrazole-3-carboxamide, tel que défini selon l'une quelconque des revendications 1 à 4 ; favorisant ainsi la formation et la précipitation de la forme A ;
- ajout de l'autre moitié de la quantité définie dudit acide chlorhydrique pendant une période de 30 min à 5 h ;
- agitation pendant une durée définie de 30 min à 2 h, de préférence de 30 min à 1 h ;
- ajout d'eau en une quantité de 5 à 15 volumes de réaction, de préférence environ 10 volumes de réaction, sur une durée de 1 - 20 h, de préférence sur une durée de 2 - 10 h ;
- éventuellement isolement de la forme A précipitée, de préférence par filtration, tamisage ou évaporation de solvant.

9. Composition pharmaceutique comprenant au moins un composé selon l'une quelconque des revendications 1 à 7, conjointement avec au moins un support, excipient et/ou diluant pharmaceutiquement acceptable.

10. Composition pharmaceutique selon la revendication 9, comprenant en outre au moins un autre agent pharmaceutiquement actif.

11. Composé selon l'une quelconque des revendications 1 à 7 ou composition pharmaceutique selon l'une quelconque des revendications 9 à 10, pour une utilisation dans le traitement d'un trouble choisi parmi les troubles hyperprolifératifs, les troubles inflammatoires et les troubles neurodégénératifs.

12. Composé ou composition pharmaceutique pour une utilisation selon la revendication 11, dans lequel/laquelle ledit trouble hyperprolifératif est un cancer, de préférence un cancer sélectionné parmi adénocarcinome, neurinome de l'acoustique, leucémie lymphoblastique aiguë, leucémie myéloïde aiguë, carcinome corticosurrénalien, cancers liés au sida, lymphome lié au sida, cancer de l'anus, cancer de l'appendice, astrocytomes, tumeur tératoïde/rhabdoïde atypique, carcinome ampullaire, carcinome basocellulaire, cancer des voies biliaires, cancer de la vessie, cancer des os, ostéosarcome et histiocytome fibreux malin, gliome du tronc cérébral, tumeur cérébrale, tumeur tératoïde/rhabdoïde atypique du système nerveux central, craniopharyngiome, épendymoblastome, épendymome, médulloblastome, médulloépithéliome, tumeurs parenchymateuses pinéales de différenciation intermédiaire, tumeurs neuroectodermiques primitives supratentorielles et pinéoblastome, tumeurs du cerveau et de la moelle épinière, cancer du sein, tumeurs de l'ouraque, lymphome de Burkitt, tumeur carcinoïde, tumeur choroïdienne mélanome, cancer gastro-intestinal, lymphome du système nerveux central, cancer du col de l'utérus, cancer du corps utérin, chordome, leucémie lymphoïde chronique, leucémie myéloïde chronique, syndromes myéloprolifératifs chroniques, cancer du côlon, cancer colorectal, lymphome cutané à cellules T, tumeur desmoïde, mycosis fongoïde, cancer de l'endomètre, cancer de l'œsophage, esthésioneuroblastome, famille des tumeurs du sarcome d'Ewing, tumeur germinale extracrânienne, tumeur germinale extragonadique, cancer des voies biliaires extrahépatiques, tumeurs de l'oreille, mélanome intraoculaire, rétinoblastome, cancer de la vésicule biliaire, cancer de l'estomac, tumeur carcinoïde gastro-intestinale, tumeur stromale gastro-intestinale, tumeur à cellules stromales gastro-intestinales, tumeurs gynécologiques, tumeur de cellules germinales de l'ovaire, tumeur trophoblastique gestationnelle, gliome, carcinomes de la vésicule biliaire, leucémie à tricholeucocytes, cancer de la tête et du cou, cancer du cœur, cancer hépatocellulaire, histiocytose, cancer de l'hypopharynx, néoplasies hématologiques, tumeurs des cellules des îlots de Langerhans (pancréas endocrine), cancer de cellules rénales, cancer du rein, histiocytose langerhansienne, cancer du larynx, leucémie, cancer des lèvres et de la cavité buccale, cancer du foie, cancer du poumon, cancer du poumon non à petites cellules, tumeurs de l'intestin grêle, cancer du poumon à petites cellules, lymphome de Hodgkin, lymphome non hodgkinien, lymphome primitif du système nerveux central, macroglobulinémie, histiocytome fibreux malin osseux et ostéosarcome, mélanome, carcinome à cellules de Merkel, mésothéliome, cancer épidermoïde métastatique avec primitifs occultes, tumeurs de la moelle épinière, syndromes de néoplasie endocrinienne multiple, syndromes myélodysplasiques, néoplasies myélodysplasiques/myéloprolifératives, leucémie myéloïde, myélome multiple, troubles myéloprolifératifs, cancer des fosses nasales et des sinus paranasaux, cancer du nasopharynx, neuroblastome, cancer de la cavité buccale, cancer de l'oropharynx, ostéosarcome et histiocytome fibreux malin osseux, cancer de l'ovaire, cancer épithélial de l'ovaire, tumeur ovarienne à faible potentiel de malignité, oligodendrogliome, plasmocytomes, cancer du pancréas, papillomatose, cancer de la parathyroïde, cancer du pénis, cancer du pharynx, tumeur hypophysaire, néoplasie plasmocytaire/myélome multiple, blastome pleuropulmonaire, cancer de la prostate, cancer du rectum, cancer de cellules rénales, cancer à cellules transitionnelles, cancer des voies respiratoires, rhabdomyosarcome, cancer des glandes salivaires, sarcome, cancer de la peau et des testicules, sarcome d'Ewing, sarcome de Kaposi, sarcome utérin, cancer de la peau non mélanome, cancer de la peau mélanome, carcinome cutané, cancer de l'intestin grêle, sarcome des tissus mous, carcinome épidermoïde, cancer épidermoïde du cou, cancer de l'estomac, tumeurs des tissus mous, cancer du testicule, cancer de la gorge, thymome et carcinome thymique, cancer de la thyroïde, cancer à cellules transitionnelles du bassinet et de l'uretère, tumeur trophoblastique, cancer du testicule, cancer gestationnel, tumeurs urologiques, cancer de l'uretère et du bassinet, cancer de l'urètre, carcinome urothélial, cancer de l'utérus, cancer du vagin, cancer de la vulve, macroglobulinémie de Waldenström et tumeur de Wilms, tumeurs qui provoquent des épanchements dans les espaces potentiels du corps, des épanchements pleuraux, des épanchements péricardiques, des épanchements péritonéaux également appelés ascite, tumeurs à cellules géantes (TCG), TCG osseuses, synovite villonodulaire pigmentée (SVNP), tumeur à cellules géantes ténosynoviale (TCGT), TCGT de la gaine tendineuse (TCGT-GT).

13. Composé ou composition pharmaceutique pour une utilisation selon la revendication 11, dans lequel/laquelle ledit trouble inflammatoire est choisi parmi arthrose, syndrome inflammatoire de l'intestin, rejet de greffe, lupus érythémateux systémique, colite ulcéreuse, maladie de Crohn, bronchopneumopathie chronique obstructive, emphysème, maladie de Kawasaki, syndrome hémophagocytaire (syndrome d'activation macrophagique), réticulohistiocytose multicentrique, athérosclérose, sclérose en plaques progressive primaire, diabète tenpsy de type 1, diabète de type 2, résistance à l'insuline, hyperglycémie, obésité, lipolyse, hypocréosinophilie, ostéoporose, risque accru de fracture, maladie de Paget, hypercalcémie, ostéolyse d'origine infectieuse (par exemple, ostéomyélite), ostéolyse périprothétique ou due aux débris d'usure, endométriose, douleur inflammatoire, douleur chronique et douleur osseuse.

14. Composé ou composition pharmaceutique pour une utilisation selon la revendication 11, dans lequel/laquelle ledit trouble neurodégénératif est choisi parmi démence de type Binswanger, prosencéphalie, microcéphalie, paralysie cérébrale, hydrocéphalie congénitale, hydropisie abdominale, paralysie supranucléaire progressive, glaucome, maladie de Wilson, maladie d'Alzheimer et autres démences, maladie de Parkinson (MP) et troubles liés à la MP, démence multi-infarctus, démence fronto-temporale, pseudo-démence, maladie à prions, maladies des motoneurones, maladie de Huntington, ataxie spinocérébelleuse et amyotrophie spinale.

15. Composé ou composition pharmaceutique pour une utilisation selon l'une quelconque des revendications 11 à 14, dans lequel/laquelle ladite utilisation est en combinaison avec un autre médicament pharmaceutiquement actif ou une autre thérapie, en particulier une radiothérapie, des agents chimiothérapeutiques, des médicaments ciblés et des médicaments inhibiteurs de point de contrôle immunitaire.
